# EUROPÄISCHE PATENTSCHRIFT

(11) **EP 0 524 143 B1**
(45) Veröffentlichungstag und Bekanntmachung des Hinweises auf die Patenterteilung: **10.12.1997**
(21) Anmeldenummer: 92810522.0
(22) Anmeldetag: 08.07.1992
(51) Int. Cl.: C12P 19/30, C12N 1/20

(54) **Verfahren zur Herstellung aktivierter Sialinsäuren**
Process of preparation of activated sialic acids
Procédé de préparation d'acides sialiques activés

(30) Priorität: 17.07.1991 CH 2119/91
(43) Veröffentlichungstag der Anmeldung: 20.01.1993
(73) Patentinhaber: Novartis AG, 4058 Basel (CH); FORSCHUNGSZENTRUM JÜLICH GMBH, 52425 Jülich (DE)
(72) Erfinder: Kittelmann, Matthias, Dr., W-7800 Freiburg (DE); Ghisalba, Oreste, Dr., W-4153 Reinach (CH); Klein, Teresa, W-5170 Jülich (DE); Kragl, Udo, Nigawa, Greenheight 105 (JP); Wandrey, Christian, Prof. Dr., W-5170 Jülich (DE)

(56) Entgegenhaltungen:
- EP-A- 0 120 328

## Beschreibung

Die Erfindung betrifft ein neues Verfahren zur Herstellung aktivierter Sialinsäuren, insbesondere CMP-aktivierter Sialinsäuren, unter Verwendung mikrobieller Zellextrakte.

Sialinsäuren spielen als wesentliche Bestandteile von Glykokonjugaten, wie Glykoproteinen und Glykolipiden, eine herausragende Rolle in einer Vielzahl von biologischen Prozessen. So sind an der Zelloberfläche exponierte Sialyloligosaccharide Rezeptordeterminanten für Viren, Mykoplasmen, pflanzliche und tierische Lektine, bakterielle Toxine, Interferone sowie gewisse Tumor- und Blutgruppen-spezifische Antikörper. Darüberhinaus erfüllen terminale Sialylreste oft eine maskierende Funktion für die von ihnen terminierten Glykanreste, so dass die An- oder Abwesenheit von terminalen Sialylresten einen entscheidenden Einfluss auf die biologische Halbwertszeit des Glykokonjugats haben kann. Beispielsweise kann man durch enzymatischen Abbau der mannosereichen N-Glykanketten von rekombinanten Hefeproteinen (z.B. rekombinantes t-PA aus S.cerevisiae) und Anhängen einer terminalen Galactosyl-Sialinsäure-Gruppe an derart gestutzte Proteine eine erheblich verlängerte Halbwertszeit erreichen.

Es sind etwa 30 verschiedene in der Natur vorkommende Sialinsäuren bekannt, von denen die N-Acetylneuraminsäure (Neu5Ac) die wichtigste darstellt. Die Biosynthese von sialylierten Glykokonjugaten erfolgt, wie eingehende Untersuchungen gezeigt haben, über Nucleotidphosphat-aktivierte Sialinsäuren, insbesondere Cytidin-5'-monophosphat (CMP)-aktivierte Sialinsäuren, welche in Gegenwart spezifischer Sialyltransferasen an die wachsende Kohlenhydratkette angehängt werden. Aus der Literatur sind zahlreiche Synthesen Nucleotidphosphat-aktivierter Sialinsäuren, wie insbesondere CMP-Neu5Ac, bekannt geworden. So verwenden Vann et al. (J.Biol.Chem.(1987) 262, 17556-17562) und Warren et al. (ibid. (1962) 237,3527-3534) mehreren Reinigungsschritten unterworfene Enzympräparationen aus E.coli beziehungsweise N.meningitidis, enthaltend die entsprechende bakterielle CMP-Neu5Ac-Synthetase, um CMP-Neu5Ac aus Cytidintriphoshat (CTP) und Neu5Ac herzustellen, während neuerdings Ichikawa et al. (J.Am.Chem.Soc. (1991) 113,4698-4700) und Shames et al. (Glycobiology (1991) Vol. 1, No. 2, 187-191) mittels rekombinanter Gentechnikverfahren hergestellte und mit Antikörper-Affinitätschromatographie bzw. Farbstoffliganden-Affinitätschromatographie gereinigte E.coli-CMP-Neu5Ac-Synthetase benutzen. Bei den gängigeren Verfahren werden Enzympräparationen tierischen Ursprungs (z.B. Schwein, Pferd, Kalb, Rind, Frosch) verwendet, vgl. z.B. Higa et al. (J.Biol.Chem. (1985) 260, 8838-8849), Thiem et al. (Liebigs Ann.Chem. (1990),1101-1105), Gross et al. (Eur.J.Biochem. (1987) 168, 595-602), Deutsche Offenlegungsschrift Nr. 3626915, um CTP und Neu5Ac zu CMP-Neu5Ac umzusetzen. Bei weiteren aus der Literatur bekannten Verfahren werden die Edukte CTP und/oder Neu5Ac auch teilweise in situ hergestellt und in Anwesenheit von CMP-Neu5Ac-Synthetase zu CMP-Neu5Ac weiter umgesetzt, so z.B. CTP aus CMP und ATP, Phosphoenolpyruvat bzw. Hefezellen, welche CMP in CTP umzuwandeln vermögen (vgl. Simon et al. J.Am.Chem.Soc.(1988) 110, 7159-7163, Simon et al. Methods Enzym. (1989) 179, 275-287, Auge et al. Tetrahedron Lett. (1988) 29, 789-790, Japanische Offenlegungsschrift Nr. 2177891), und Neu5Ac aus N-Acetylglucosamin bzw. N-Acetylmannosamin und Pyruvat (vgl. Simon et al. (1988,1989), loc.cit.). EP-A-0 120 328 beschreibt ebenfalls eine enzymatische Methode zur Herstellung von CMP-aktivierter Sialinsäure aus CTP und Neu5Ac. Darin wird entweder gereinigte CMP-Neu5Ac Synthetase aus tierischen Geweben für die in vitro Synthese oder CMP-Neu5Ac Synthetase in E. coli CRC-1482 für die in vivo Synthese von CMP-Neu5Ac eingesetzt. Bezogen auf Neu5Ac muss CTP in vielfachem molaren Überschuss eingesetzt werden. Ausserdem sind dem Reaktionsansatz Stabilisatoren zuzusetzen.

Den bekannten Verfahren haftet der Nachteil an, dass einerseits zur Herstellung tierischer Enzympräparate aufwendige und kostspielige Reinigungsverfahren herangezogen werden müssen und in Zellhomogenaten tierischer Gewebe die CMP-Neu5Ac-Synthetase nur in äusserst geringen Mengen vorliegt, so dass grosse Volumina an Biomasse aufgearbeitet werden müssen, um das Enzym in den für präparative Zwecke notwendigen Mengen zu gewinnen. Darüberhinaus ist es bei Verwendung tierischer Gewebe als Enzymquelle nicht gewährleistet, dass diese zu jeder Zeit in grossen Mengen mit gleichbleibend hoher Qualität zu Verfügung stehen und frei von viralen Kontaminationen sind, welche ihre Verwendbarkeit in Frage stellen würden. Es besteht daher ein Bedürfnis nach neuen Verfahren zur Herstellung von CMP-Neu5Ac-Synthetase, welche es erlauben, dieses Enzym in beliebiger Menge und gleichbleibend hoher Qualität, insbesondere ohne den aus tierischen Quellen anhaftenden Nachteilen und Risiken, zu gewinnen.

Es wurde jetzt überraschenderweise gefunden, dass sich aus natürlich vorkommenden Mikroorganismen mit Aktivität an Cytidin-5'-monophospho-N-acetylneuraminsäureSynthetase (CMP-Neu5Ac-Synthetase) Zellrohextrakte gewinnen lassen, die ohne weitere Reinigung oder nach lediglich einem vorgeschalteten Reinigungsschritt die Umsetzung von CTP mit Neuraminsäuren zu den entsprechenden CMP-aktivierten Neuraminsäuren katalysieren. Die erfindungsgemässen Zellextrakte sind in beliebigen Mengen verfügbar, da grosse Volumina an Biomasse mit gleichbleibend hoher Enzymaktivität unter standardisierten Bedingungen auf einfache Weise durch Fermentation gewonnen werden können.

Die Erfindung betrifft demgemäss ein Verfahren zur Herstellung von Cytidin-5'-monophospho-sialinsäuren, dadurch gekennzeichnet, dass man eine Sialinsäure mit Cytidin-5'- triphosphat in Gegenwart eines gegebenenfalls einem Reinigungsschritt unterworfenen Zellextraktes eines natürlich vorkommenden Mikroorganismus mit Aktivität an Cytidin-5'-monophospho-N-acetylneuraminsäure-Synthetase umsetzt.

Geeignete Sialinsäuren gemäss vorliegender Erfindung sind beispielsweise die bei Reuter et al. ( Proceedings of the Japanese-German Symposium on Sialic Acids (1988), 98-100) genannten. Insbesondere kommen die folgenden Sialinsäuren in Betracht: N-Acetylneuraminsäure (Neu5Ac), N-Acetyl-4-O-acetylneuraminsäure (Neu4,5Ac₂), N-Acetyl-9-O-acetylneuraminsäure ((Neu5,9Ac₂), N-Acetyl-7,9-di-O-acetylneuraminsäure (Neu5,7,9Ac₃), N-Acetyl-8,9-di-O-acetylneuraminsäure (Neu5,8,9Ac₃), N-Acetyl-9-O-lactoylneuraminsäure (Neu4Ac9Lt), N-Acetyl-4-O-acetyl-9-O-lactoylneuraminsäure (Neu4,5Ac₂9Lt), N-Acetylneuraminsäure-9-phosphat (Neu5Ac9P), N-Glycoloylneuramin-säure (Neu5Gc), N-Glycoloyl-9-O-acetylneuraminsäure (Neu9Ac5Gc), N-Glycoloyl-9-O-lactoylneuraminsäure (Neu5Gc9Lt), N-Glycoloylneuraminsäure-8-sulfat ((Neu5Gc8S), ferner 5-Azidoneuraminsäure, N-Acetyl-9-azido-9-deoxy-neuraminsäure und N-Acetyl-9-acetamido-9-deoxy-neuraminsäure. Hervorzuheben sind Neu4,5Ac₂, Neu5,9Ac₂, Neu5Gc, Neu9Ac5Gc, N-Acetyl-9-acetamido-9-deoxy-neuraminsäure, N-Acetyl-9-azido-9-deoxy-neuraminsäure, 5-Azidoneuraminsäure und in erster Linie Neu5Ac.

Die genannten Sialinsäure-Verbindungen können als solche in die Reaktion eingesetzt werden oder aber im Reaktionsgemisch aus geeigneten Vorstufen in situ hergestellt werden. So kann beispielsweise Neu5Ac in situ aus N-Acetylmannosamin (Man2Ac) und Pyruvat, wobei dem Reaktionsgemisch zusätzlich eine Neu5Ac-Aldolase zugesetzt werden muss, hergestellt werden. Weiterhin kann z.B. anstelle von Neu5Ac auch N-Acetylglucosamin, welches mit Pyruvat in Gegenwart einer N-Acetyl-glucosamin-Epimerase und der genannten Aldolase über Man2Ac in situ in Neu5Ac überführt wird, in die Reaktion eingesetzt werden.

Cytidin-5'-triphosphat (CTP) kann gleichfalls als solches oder in Form einer in CTP überführbaren Verbindung in die Reaktion eingesetzt werden. Geeignete Vorstufen sind beispielsweise Cytidin-5'-diphosphat (CDP) oder insbesondere Cytidin-5'-monophosphat (CMP), welche mit ATP oder Phosphoenolpyruvat in Gegenwart geeigneter Enzyme in CTP überführt werden. Da die zur Ueberführung von CMP bzw. CDP in CTP benötigten Enzyme in den erfindungsgemäss verwendeten mikrobiellen Rohextrakten bereits enthalten sind, erübrigt sich die Zugabe exogener Enzyme (bei den in der Literatur beschriebenen Verfahren, z.B. Thiem et al., Auge et al., Simon et al. (1988, 1989), loc. cit) wird z.T. ebenfalls CTP in situ generiert, es werden aber käufliche Enzyme eingesetzt).

Erfindungsgemäss verwendbare Zellextrakte von natürlich vorkommenden Mikroorganismen mit CMP-Neu5Ac-Synthetase-Aktivität sind insbesondere solche von Escherichia coli Serotyp K1, Spezies von Streptococcus Serotyp B oder C, Neisseria meningitidis, Pasteurella haemolytica, Pasteurella multacida, Moraxella nonliquefaciens, Citrobacter freundii, Salmonella-Spezies, z.B. S. dahlem, S.- djakarta oder S. trouca, Actinomyces viscosus, ferner Corynebacterium parvum (Propionibacterium acnes). Grundsätzlich können Zellextrakte aller Mikroorganismen mit CMP-Neu5Ac-Synthetase-Aktivität verwendet werden. Bevorzugt sind Zellextrakte von E.coli-Stämmen des Serotyps K1, in erster Linie E.coli K-235 (ATCC 13027).

Derartige natürlich vorkommende Mikroorganismen mit CMP-Neu5Ac-Synthetase-Aktivität können in an sich bekannter Weise durch Mutation, beispielsweise unter Verwendung allgemein bekannter Mutagene, wie UV- oder Röntgen-Strahlen bzw. mutagene Chemikalien, in Mutanten überführt werden, die sich von den Eltern durch verbesserte Eigenschaften, z.B. geringere Nährmediumansprüche, grössere Wachstumsraten und insbesondere höhere Produktivität an CMP-Neu5Ac-Synthetase, unterscheiden. Derartige Mutanten können auch spontan auftreten. Die Identifizierung und Isolierung solcher Mutanten erfolgt ebenfalls in an sich bekannter Weise: die CMP-Neu5Ac-Synthetase-Aktivität von Kolonien derartiger Mutanten wird beispielsweise nach dem Zellaufschluss ermittelt, in dem man aliquote Teile des Zellüberstandes mit definierten Mengen an CTP und Neu5Ac versetzt und das gebildete CMP-Neu5Ac chromatographisch, insbesondere mittels HPLC, qualitativ oder quantitativ bestimmt. Besonders bevorzugte Mutanten sind solche von E.coli K-235 mit erhöhter Produktivität an CMP-Neu5Ac-Synthetase und/oder verminderter Schleimbildung. Eine solche E.coli K-235/CS1 genannte, spontan auftretende Mutante wurde als Satellitenkolonie von grossflächig wachsenden, schleimigen E.coli K-235-Kolonien isoliert. Die neue Mutante unterscheidet sich wie folgt vom Herkunftsorganismus K-235: 1) Koloniemorphologie, 2) höhere Wachstumsgeschwindigkeit im Verlauf der Fermentation und 3) höhere spezifische Aktivität in Schüttelkultur sowie in der Fermentation, während die Zellmorphologie (mikroskopishes Bild) sowie das Spektrum der verwertbaren Kohlenstoffquellen identisch sind. Der neue Stamm E.coli K-235/CS1 ist ebenfalls ein Gegenstand der vorliegenden Erfindung.

Die Umsetzung der Sialinsäuren, z.B. Neu5Ac, mit CTP in Gegenwart des mikrobiellen Zellextraktes wird bevorzugt in homogener wässriger Lösung in Gegenwart von etwa 10-50, insbesondere etwa 20-40 mM Mg²⁺, z.B. in Form eines entsprechenden Halogenids oder Sulfats, z.B. MgCl₂ oder MgSO₄, oder in Gegenwart von etwa 5-30, insbesondere etwa 10-15 mM Mn²⁺, z.B. in Form eines entsprechenden Halogenids oder Sulfats, wie MnCl₂ oder MnSO₄, durchgeführt. Der pH-Wert der Reaktionsmischung wird bei Anwesenheit von Mg-Ionen auf etwa 8-11, bevorzugt 8-10, und bei Anwesenheit von Mn-Ionen auf etwa 6-8, bevorzugt 7,5 eingestellt, wobei man zur Stabilisierung des pH-Wertes in an sich bekannter Weise in gepufferter Lösung oder unter Verwendung eines pH-Staten arbeitet. Die Umsetzungstemperatur liegt etwa bei 20-35°C, bevorzugt bei etwa 25-30°C. Die Edukte werden bevorzugt in äquimolaren Mengen eingesetzt.

Bei der in situ-Generierung von CTP verfährt man in analoger Weise wie oben beschrieben, ausser dass man anstelle von CTP eine äquimolare Menge an CMP (bzw. CDP) und ATP oder Phosphoenolpyruvat (PEP) einsetzt, wobei ATP und PEP gegebenenfalls auch im Ueberschuss, z.B. im zwei- bis dreifachen Ueberschuss, verwendet werden können.

Die in situ-Generierung von Neu5Ac aus N-Acetylmannosamin (Man2Ac) und Pyruvat wird in Gegenwart einer Neu5Ac-Aldolase durchgeführt. Da das pH-Optimum der Aldolase im neutralen Bereich liegt, wird bei pH 6-8, bevorzugt pH 7-7,5 gearbeitet, weshalb die Verwendung von Mn²⁺-Salzen bei der anschliessend ablaufenden Aktivierung des entstandenen Neu5Ac bevorzugt ist. Entsprechend wird bei der in situ-Generierung von Neu5Ac aus N-Acetylglucosamin und Pyruvat ebenfalls im neutralen Bereich gearbeitet, beispielsweise bei pH 6-8, bevorzugt bei pH 7-7,5, da sowohl die zuzusetzende N-Acetyl-glucosamin-Epimerase, welche die Ueberführung des N-Acetylglucosamins in N-Acetylmannosamin katalysiert, als auch, wie oben beschrieben, die Neu5Ac-Aldolase ihre Wirkungsoptima im neutralen Bereich aufweisen. Somit wird auch im zuletzt genannten Fall bevorzugt in Gegenwart von Mn²⁺-Salzen gearbeitet, um optimale Bedingungen für die anschliessend ablaufende Aktivierung des gebildeten Neu5Ac vorzugeben.

Die Aktivierung der Sialinsäuren, z.B.Neu5Ac, durch CTP mit Hilfe mikrobieller Enzyme gemäss vorliegender Erfindung kann auch in Gegenwart einer Pyrophosphatase durchgeführt werden, welche das neben dem gewünschten Hauptprodukt, CMP-aktivierte Sialinsäure, anfallende Nebenprodukt der Reaktion, das Pyrophosphat, durch Hydrolyse in zwei Aequivalente Phosphat aus dem Reaktionsgleichgewicht entfernt. Auf diese Weise kann die Gleichgewichtslage der Reaktion weiter auf die Produktseite verschoben werden, was insbesonders bei Anwendung der gekoppelten Enzymsysteme bestehend aus Neu5Ac-Aldolase und CMP-Neu5Ac-Synthetase bzw. aus N-Acetylglucosamin-Epimerase und oben genannter Aldolase und Synthetase von Vorteil ist.

Das erfindungsgemässe Verfahren kann sowohl im Batch-Verfahren als auch kontinuierlich im Enzymmembranreaktor (EMR) durchgeführt werden. Dabei ist der Enzymmembranreaktor vorzugsweise mit einer Ultrafiltrationsmembran mit einem Abscheidungsgrad kleiner als ca. 30000 ausgestattet, so dass die im Reaktionsgemisch enthaltenen Enzyme zurückgehalten werden, während das niedermolekulare Produkt (CMP-aktivierte Sialinsäure, z.B.CMP-Neu5Ac) und nicht umgesetzte Edukte die Membran passieren und das Produkt aus dem Auslauf isoliert werden kann. Vorzugsweise wird der Reaktor vor Gebrauch sterilisiert, so dass auf den Zusatz antibakterieller Substanzen verzichtet werden kann. Die Umsetzungen erfolgen in analoger Weise wie oben beschrieben, wobei man aus den genannten Gründen bei der Verwendung von beispielsweise Neu5Ac als Ausgangsmaterial sowohl Mn²⁺- als auch Mg²⁺-Salze unter gleichzeitiger Berücksichtigung des optimalen pH-Wertes anwenden kann, während bei der in situ-Herstellung von Neu5Ac aus N-Acetylmannosamin bzw. N-Acetylglucosamin bei entsprechendem pH-Wert bevorzugt Mn²⁺-Salze in Frage kommen. Zweckmässig ist bei der kontinuierlichen Produktion von CMP-aktivierten Sialinsäuren im EMR eine pH-Kontrolle am Auslauf (pH-Sonde) sowie eine pH-Regelung (pH-Stat) am Einlauf (Zudosieren von NaOH) eingebaut, welche das entstehende Pyrophosphat neutralisiert. Im übrigen können die Parameter für die Umsetzung im Enzymmembranreaktor so bestimmt und gewählt werden, wie für die Herstellung von Neu5Ac im EMR gemäss Deutscher Offenlegungsschrift Nr. 3937891 beschrieben.

Das erfindungsgemässe Verfahren kann auch durch Perkolation der auf einen geeigneten pH-Wert eingestellten Lösung, enthaltend die Edukte CTP und Sialinsäure, über ein festes Trägermaterial, an das die im mikrobiellen Rohextrakt enthaltene CMP-Neu5Ac-Synthetase immobilisiert wurde (das matrix-gebundene Enzympräparat ist beispielsweise durch Perkolation des mikrobiellen Rohextraktes über CNBr-aktivierte Sepharose, Eupergit und dergleichen erhältlich), durchgeführt werden.

Die Aufarbeitung des Reaktionsgemisches und die Reinigung der erfindungsgemäss hergestellten CMP-aktivierten Sialinsäuren erfolgt mit üblichen aus dem Stand der Technik bekannten Verfahren. Beispielsweise kann die Reaktionsmischung durch Filtration oder vorzugsweise Zentrifugation geklärt und danach durch Ultrafiltration (Membran mit Abscheidungsgrad ≤30000 Dalton) das Enzym abgetrennt und restliches Produkt aus dem Retentat durch Diafiltration ausgewaschen werden.Die eigentliche Aufreinigung erfolgt dann z.B. mit chromatographischen Methoden, z.B. Gelchromatographie (u.a. Sephadex G-25), Ionenaustausch-, z.B. Anionenaustauschchromatographie, Dünnschichtchromatographie, HPLC und dergleichen.Das Reinigungsverfahren kann erheblich vereinfacht werden, wenn man die nach Abschluss der Reaktion noch im Reaktionsgemisch verbliebenen Cytidinnucleotide (CMP, CDP und CTP) mit alkalischer Phosphatase dephosphoryliert.

Zur Gewinnung des verfahrensgemäss verwendeten Zellextraktes wird ein natürlich vorkommender Mikroorganismus mit Aktivität an Cytidin-5'-monophospho-N-acetylneuraminsäure-Synthetase, insbesondere einer der oben genannten, in einem wässrigen Nährmedium, das assimilierbare Kohlenstoff- und Stickstoff-Quellen sowie Mineralsalze enthält, bei einem pH-Wert zwischen etwa 6 und 9, bevorzugt bei etwa 6,5-7, und bei einer Temperatur von etwa 33-40°C, insbesondere bei etwa 37°C, kultiviert, die Zellmasse abgetrennt und der Zellextrakt gewonnen. Die Kultivierung der genannten Mikroorganismen erfolgt aerob (z.B. im Schüttelkolben), ausser im Falle von Corynebacterium parvum, welches nur anaerob oder bei sehr geringen Sauerstoffpartialdrücken zur Vermehrung befähigt ist, z.B. bei Bebrütung in hoher Schicht unter Luftabschluss ohne Rühren. Die Fermentationszeit wird so gewählt, dass optimale Titer bezüglich CMP-Neu5Ac-Synthetase-Aktivität erreicht werden.

Für eine erfolgreiche Anzucht der geeigneten Mikroorganismen ist eine gute Belüftung (ausser bei C.parvum, s.o.), ferner auch ein stufenweises Nachdosieren ("fed batch") der Nährstoffe wichtig. Als Kohlenstoffquelle kommen insbesondere einfache organische Säuren, wie Essigsäure, Aepfelsäure,Milchsäure oder Bernsteinsäure, ferner Zucker, wie insbesondere Glucose oder Sorbit, in Betracht. Geeignete Stickstoffquellen sind entsprechende Mineralsalze, wie Ammoniumsalze anorganischer Säuren, z.B. Ammoniumchlorid oder -sulfat (z.B. bei E.coli und Salmonella-Spezies), ferner auch stickstoffhaltige komplexe Mischungen, wie Fleischextrakt, Hefeextrakt, Caseinhydrolysat, Maisquellwasser, Brain Heart Infusion, Trypticase Soy Broth usw., einzeln oder in Kombination. Ferner enthält das Nährmedium eine Quelle für Wuchsstoffe, wie Vitamine, Purine, Pyrimidine, Aminosäuren und Spurenelemente, bevorzugt in komplexer Form (enthalten z.B. in den oben erwähnten komplexen stickstoffhaltigen Mischungen). Bei einigen erfindungsgemäss verwendbaren Mikroorganismen, z.B. E.coli, können auch Mineralsalzmedien mit einer organischen Kohlenstoffquelle, z.B. eine der oben genannten, zur Anwendung gelangen (gegebenenfalls unter Zusatz von definierten Wuchsstoffen). Einige pathogene Mikroorganismen, wie Moraxella spec. und Pasteurella spec., benötigen zu gutem Wachstum zusätzlich die Anwesenheit von Hämin oder anderer Faktoren, die in Form von Blut angeboten werden können. Es ist zweckmässig, die optimalen Kultivationsparameter für den jeweiligen verwendeten Mikroorganismus in an sich bekannter Weise experimentell zu ermitteln.

Wenn die Zelldichte einen ausreichenden Wert erreicht hat, wird die Kultivierung abgebrochen. Die Kulturbrühe wird in bekannter Weise, z.B. durch Zentrifugation, abgetrennt und die sedimentierten Zellen werden in üblicher Weise, z.B. durch Schütteln mit feinen Glasperlen, Ultraschallbehandlung oder Frenchpresse, aufgeschlossen. Unlösliche Zellbestandteile und, falls verwendet, Glasperlen werden z.B. durch Zentrifugation entfernt und der Ueberstand als Enzymquelle (Rohextrakt) verwendet. Der Ueberstand kann als CMP-Neu5Ac-Synthetase-haltiger Rohextrakt direkt in das erfindungsgemässe Verfahren eingesetzt werden. Zweckmässigerweise wird der Rohextrakt zur Entfernung der Nukleinsäuren (viskose Lösungen!) jedoch mit einem polykationischen Agenz, z.B. Polyethylenimin, Polyamine wie Spermidin, Streptomycin-Sulfat, ferner Ribonuclease oder Mn²⁺-Salze, behandelt und die präzipitierten Nukleinsäuren durch Zentrifugation entfernt.

Die Abtrennung der Zelltrümmer vom Zellhomogenat kann auch mittels wäßriger Zweiphasen-Systeme erfolgen. Dazu werden Polymere wie Polyethylenglycol (PEG) mit Molekulargewichten (MG) von 400 bis 20000 oder Dextran, insbesondere aber PEG mit MG von 400 bis 4000, mit Salzen, wie Natrium- oder Kaliumcitrat, bzw. Natrium- oder Kaliumphosphat, und mit dem aufgeschlossenen Zellhomogenat aus Escherichia coli K-235 versetzt. Die drei Komponenten werden in den unter angegebenen Mengen gemischt und/oder geschüttelt. Nach dem Abzentrifugieren entstehen zwei Phasen: eine obere PEG-angereicherte Phase, in der sich der größte Teil des Enzyms befindet und eine untere salzreiche Phase, in der sich Zelltrümmer, Nucleinsäuren, Begleitprotein und andere feste Bestandteile anreichern. Die bevorzugten erfindungsgemäss verwendbaren Zweiphasen-Systeme setzen sich wie folgt zusammen:
- PEG (MG 1550 bis 4000) mit Anteilen von 16 bis 20 w/v;
- Salze wie Natriumcitrat oder Kaliumphosphat in 9-12 w/v;
- pH-Wert bei der Extraktion ca. 7,0 bis 8,5;
- Extraktionstemperatur ca. 10-20°C.
- Gegebenenfalls kann mit einem zweiten Phasensystem eine Rückextraktion durchgeführt werden (bei Verwendung von E. coli K-235 als Zellhomogenat nicht erforderlich). Diese dient der Rückgewinnung der Synthetase aus der PEG-Phase des ersten Phasensystems in die Salzphase.

Die Abtrennung der Synthetase von den anderen Zellbestandteilen mittels wäßriger Zweiphasen-Systeme führt zu der gleichzeitigen Abtrennung der Phosphatasen sowie der CTP-asen und Nucleotidasen, die sich bei den bisherigen Synthetase-Aufreinigungsmethoden mitunter als störend bemerkbar gemacht haben. Die Phosphatase-Aktivität kann mit einem Test überprüft werden. Dazu wird die Enzymprobe mit dem Substrat p-Nitrophenylphosphat (PNPP) versetzt und bei 30°C inkubiert. Wenn Phosphatasen vorhanden sind, spalten sie die Phosphatgruppe der Nitroverbindung, so daß das gelbe p-Nitrobenzol entsteht. Die Zunahme der Gelbfärbung wird photometrisch bei 405 nm verfolgt und ist proportional der Menge an vorhandenen Phosphatasen in der Probe.

Vorzugsweise wird der Zellrohextrakt einem Reinigungsschritt unterworfen, um störende Bestandteile im Extrakt, wie z.B. CTP- und CMP-Neu5Achydrolysierende Enzyme (bei Nichtanwendung des wässrigen Zweiphasensystems, s.o.) und Neuraminsäurealdolasen, abzutrennen. Es wurde überraschenderweise gefunden, dass ein konventioneller Reinigungsschritt ausreicht, um einen für das erfindungsgemässe Verfahren hervorragend geeigneten Zellextrakt zu erhalten.

Beispielsweise kann ein für die Umsetzung von Sialinsäuren,z.B. Neu5Ac, mit CTP geeignetes CMP-Neu5Ac-Synthetase-Präparat erhalten werden, in dem man letzteres mit Polyethylenglykol (PEG) ausfällt, wobei das PEG vorzugsweise ein Molekulargewicht von etwa 200-20000, insbesondere 400-6000, aufweist und in einer Konzentration von etwa 10-50% (w/v), insbesondere 15-40% (w/v) eingesetzt wird. Alternative Reinigungsschritte im Batch-Verfahren stellen beispielsweise die Anionenaustauscherchromatographie (batchweise Adsorption bei niedriger Salzkonzentration und Desorption bei höherer Salzkonzentration, z.B. 200-300 mM NaCl), z.B. an DEAE-Sephacel, DEAE-Sepharose, Q-Sepharose, QAE-Sephadex, Mono-Q und DEAE-Biogel, und die hydrophobe Interaktionschromatographie (HIC, Adsorption im Batch-Verfahren mit hoher Salzkonzentration, z.B. 30% Ammoniumsulfat, Desorption bei niedriger Salzkonzentration), z.B. an Phenyl-, Octyl- oder Butylsepharose, ferner Butyl-, Hexyl-, Octylagarose, Alkylsuperose und Butylfraktogel usw., dar. Insbesondere durch Batch-Adsorption an Anionenaustauschern oder hydrophoben Gelen gereinigte Rohextrakte können auch zur in situ-Generierung von CTP während der erfindungsgemässen Herstellung der CMP-aktivierten Sialinsäuren Verwendung finden, wobei ATP bzw. Phosphoenolpyruvat als Phosphatgruppendonatoren für die zweifache Phosphorylierung von CMP zu CTP dienen. Weitere für die einfache Reinigung des Zellrohextraktes geeignete Trennmedien stellen z.B. Hydroxylapatit, die Gelfiltration, z.B. an Sephacryl, Superdex oder Bio-Gel, die Metallchelat-Affinitätschromatographie, z.B. an Chelating Sepharose oder Chelating Superose, und die Chromatofokussierung dar.

Eine besonders bevorzugte Methode der CMP-Neu5Ac-Synthetase-Aufreinigung stellt die Kombination
- Abtrennung der Zelltrümmer vom Zellhomogenat mittels wässriger Zweiphasensysteme und
- Reinigung mittels HIC
dar. Diese Synthetaseaufreinigungs-Kombination ist einfach durchzuführen. Die wäßrigen Zweiphasen-Systeme und die HIC ermöglichen auch eine problemlose Maßstabsvergrößerung bei gleichbleibender Qualität. Die Methode führt zu guten Enzymausbeuten. Das aufgereinigte Phosphatase-arme Enzym ist direkt im Enzym-Membran-Reaktor (EMR) einsetzbar.

Der gegebenenfalls einem Reinigungsschritt unterworfene, CMP-Neu5Ac-Synthetase-Aktivität aufweisende Zellrohextrakt kann, falls gewünscht, zwecks Verringerung des Lösungsvolumens einem Aufkonzentrierungsschritt unterworfen werden. Solche Aufkonzentrierungsschritte beinhalten beispielsweise die Ultrafiltration, ferner die Lyophilisation und die Proteinfällung mit Ammoniumsulfat mit anschliessender Aufnahme in einen geeigneten Puffer.

Die verfahrensgemäss verwendeten Ausgangsmaterialien, wie CMP, CTP, Sialinsäuren und die gegebenenfalls verwendeten Enzyme (Neu5Ac-Aldolase und N-Acetylglucosamin-Epimerase) sind im Handel erhältlich oder können nach in der Literatur beschriebenen Verfahren in an sich bekannter Weise hergestellt werden.

Die gemäss vorliegender Erfindung erhältlichen CMP-aktivierten Sialinsäuren können in an sich bekannter Weise als Bausteine für Glykokonjugate (z.B. Glykoproteine oder Glykolipide) verwendet werden. Solche Glykokonjugate sind insbesondere im Pharmaund Agrobereich sowie generell für das sogenannte "drug targeting" von Bedeutung. Hierbei dient die CMP-aktivierte Sialinsäure, z.B. Neu5Ac, als Substrat für Sialyltransferasen, welche die Zuckersäure auf geeignete Akzeptoren übertragen können, wie z.B. auf mit Endoglucosidase H behandelte und enzymatisch galaktosylierte Invertase, enzymatisch galaktosyliertes rek.-tPA, Ovalbumin oder chemisch modifiziertes Rinderserum-Albumin (BSA). So dient die terminale Galaktose-Neu5Ac-Gruppierung beispielsweise als Mimic der N-Glykanketten des komplexen Typus humaner Glykokonjugate. Die entsprechend sialylierten Derivate des rek.-tPA und des BSA weisen im Vergleich zu den entsprechenden Ausgangsproteinen z.B. die mittels rekombinanter Verfahren aus transformierter Hefe erhältlichen Proteine) eine verlängerte Halbwertszeit im Blutplasma auf. Gewisse CMP-aktivierte Sialinsäuren, z.B. CMP-Neu5Ac, weisen auch eine pharmakologische Wirkung auf. So unterstützt z.B. CMP-Neu5Ac die Regenerierung von Nervenzellen.

Die folgenden Beispiele dienen zur Illustration der Erfindung und sollen diese in keiner Weise einschränken.

### Beispiel 1: Optimierung der Nährlösung

### a) Variation der Kohlenstoffquelle

Als Grundlage zur Optimierung des Nährmediums für die Produktion der CMP-Neu5Ac-Synthetase aus Escherichia coli K-235 (ATCC 13027) dient die Nährlösung nachstehender Zusammensetzung, welche von Uchida et al. (Agr.Biol.Chem.(1973) 37,2105-2110) für die Gewinnung von "Colominic Acid" mit Escherichia coli entwickelte worden ist:

| | |
|---|---|
| Sorbit | 20 g/l |
| Hefeextrakt | 0.5 g/l |
| K₂HPO₄ | 14 g/l |
| (NH₄)₂SO₄ | 5 g/l |
| MgSO₄×7H₂O | 0.5 g/l |
| pH-Wert | 7.8 |

Dieser Nährlösung ohne Sorbit werden in 10 verschiedenen Ansätzen 3 Kohlenstoffquellen zu 10 g/l zugesetzt. Hierzu werden 100 ml Erlenmeyerkolben mit je 20 ml Füllstand des entsprechenden Mediums durch Autoklavieren bei 121°C für 20 min. sterilisiert und mit Zellen von Escherichia coli K-235, angezüchtet auf einem geeigneten festen Nährboden, angeimpft. Die Kulturen werden aerob bei 37°C und 220 UpM in einer Schüttelmaschine bebrütet. Nach 15-16 h wird der Inhalt der Schüttelkolben zentrifugiert (20 min., 8000 x g in einer Kühlzentrifuge), und die sedimentierten Zellen werden in einer Pufferlösung mit pH-Wert 9, bestehend aus 50 mM Tris/HCl, 1 mM Dithiothreitol und 20 mM MgCl₂, suspendiert.

Die Mikroorganismen in dieser Suspension werden durch Schütteln mit feinen Glasperlen aufgeschlossen. In einem Eppendorf-Reaktionsgefäss werden 0.6 ml Suspension mit 1.2 g Glasperlen (0.1-0.3 mm Durchmesser) versetzt und dann mittels spezieller Einsätze in einer Schwingmühle der Fa. Retsch (Haan, FRG) für 10 min. mit maximaler Geschwindigkeit geschüttelt. Unlösliche Zellbestandteile und Glasperlen werden abzentrifugiert (13000 UpM, Biofuge A, Fa. Hereus, Osterode, FRG) und der Überstand als Enzymquelle (Rohextrakt) genutzt.

Die Ansätze zur Aktivitätsbestimmung beinhalten:

| | |
|---|---|
| 1.5 M Tris/HCl Puffer, pH 9 | 25 µl |
| 0.4 M MgCl₂ | 25 µl |
| 0.01 M Dithiothreitol | 25 µl |
| 0.1 M Neu5Ac | 25 µl |
| Deionisiertes Wasser | 100 µl |
| Enzymlösung | 25 µl |

Die Enzymreaktion wird durch Zugabe von 25 µl einer 0.1 M CTP-Lösung gestartet. Zur qualitativen Analyse werden nach ca. 175 min. Inkubation bei 30°C jedem Ansatz 4 µl Probe entnommen und dünnschichtchromatographisch auf Kieselgelplatten (Typ 60 F-254, Fa. Merck, Darmstadt, FRG) aufgetrennt. Als mobile Phase dient eine Mischung aus Ethanol, 95%ig, und Ammoniumacetat, 1 M, im Verhältnis 7:3. Die Detektion erfolgt im UV-Licht bei 254 nm. Nach 3 stündiger Inkubation wird die enzymatische Reaktion durch Zusatz von 250 µl kaltem Aceton abgestoppt. Die Ansätze werden 10 min. im Eisbad gekühlt und anschliessend die Ausfällungen an Protein und Salzen durch Zentrifugation für 10 min. bei 13000 UpM in einer Biofuge A abgetrennt.

Die quantitative Bestimmung des Reaktionsproduktes CMP-Neu5Ac erfolgt mittels HPLC unter Verwendung einer Amino-Nukleosilsäule als stationäre Phase (250 x 4 mm, 10 µm Partikeldurchmesser, Fa. Innovativ Bischoff AG, Wallisellen). Das Injektionvolumen beträgt 7 µl. Eluiert wird während 8 min. an einem Gradient, bei welchem die Kaliumphosphatkonzentration (pH 7) von 3 auf 250 mM ansteigt und die Acetonitrilkonzentration von 50 auf 0 % abnimmt. Die Berechnung der CMP-Neu5Ac Konzentration erfolgt an Hand eines externen Standards (0.25 mM, CMP-Neu5Ac, Na-Salz; Sigma, Buchs, Schweiz) über die Integration der Peakfläche.

Die Enzymaktivität wird in internationalen Units angegeben, wobei eine Einheit (U) einer Menge von 1 µMol gebildeten CMP-Neu5Ac pro Minute entspricht. Mit einer spezifischen Aktivität von 1 mU/mg wird eine Umsatzrate von 1 nMol pro Minute pro mg Protein (Biorad-Proteintest, Fa. Biorad-Laboratories, Glattbrugg) bezeichnet.

Wie Tabelle 1 zeigt, weist der verwendete E.coli-Stamm bei Wachstum auf Sorbit die höchste Synthetaseaktivität auf, so dass dieser Zuckeralkohol als Kohlenstoffquelle für die Produktion des Enzyms verwendet wird.

**Tabelle 1:**

| Variation der Kohlenstoff-Quelle zur Produktion der CMP-Neu5Ac-Synthetase aus Escherichia coli K-235 | | | |
|---|---|---|---|
| C-Quelle (10 g/l) | Wachstum | spez. Aktivität (mU/mg) | Volumenausbeute (U/l) |
| Sorbit | +++ | 8.27 | 1.22 |
| Succinat | + | 1.71 | 0.04 |
| Malat | + | 2.04 | 0.06 |

### b) Variation der Stickstoffquelle

Im "Colominic Acid"-Produktionsmedium ohne Hefeextrakt (siehe Beispiel 1a) werden verschiedene komplexe Labornährstoffe und technische Nährstoffe je zu 5 g/l als Stickstoffquelle eingesetzt. Weiterhin wird o.g. Medium mit 5 g/l Hefeextrakt und ohne Ammoniumsulfat durch 8 stickstoffhaltige Reinsubstanzen ergänzt, welche in molaren Konzentrationen bezüglich des Stickstoffgehaltes entsprechend 5 g/l Ammoniumsulfat eingesetzt werden. Im übrigen wird wie unter Beispiel 1a vorgegangen.

Wie Tabelle 2 zeigt, kann mit verschiedenen komplexen und synthetischen Stickstoffquellen wie z.B. Hefeextrakt, Pepton oder Cornsteep bzw. Ammoniumsulfat, Glutamat und Asparagin eine hohe Enzymproduktion erreicht werden. Im folgenden werden Hefeextrakt in technischer Qualität auf Grund der hohen spezifischen Aktivität und Ammoniumsulfat auf Grund der hohen Volumenausbeute für die Produktion der Synthetase verwendet.

**Tabelle 2:**

| Variation der Stickstoff-Quelle im "Colominic Acid"-Produktionsmedium | | | |
|---|---|---|---|
| N-Quelle (5g/l) | Wachstum (O.D.660) | spez.Aktivität (mU/mg) | Volumenausbeute (U/l) |
| a) Versuch 1: Labor- und technische Nährstoffe, 24 h Anzucht | | | |
| Hefeextrakt (Fluka) | +++ | 12.9 | 1.84 |
| Fleischextrakt | +++ | n.b. | 1.69 |
| Lab Lemco Pulver | ++ | 5.14 | 0.89 |
| Pepton, trypt. verdaut | +++ | 11.1 | 1.67 |
| Fischpepton FPH-30 | +++ | 2.76 | 0.37 |
| Fischpepton H-0100 | +++ | 10.0 | 1.56 |

| b) Versuch 2: komplexe technische Nährstoffe, 16 h Anzucht | | | |
|---|---|---|---|
| ohne | 0.55 | 0.0 | 0.0 |
| Hefeextrakt (Fluka) | 3.90 | 12.21 | 4.51 |
| Hefeextrakt, techn. | 6.28 | 15.4 | 4.77 |
| Casamino Acids | 4.13 | 14.0 | 4.77 |
| Caseinhyrolysat | 5.30 | 11.5 | 4.03 |
| Cornsteep | 7.15 | 14.9 | 4.77 |
| Erdnussmehl | 5.7 | 10.6 | 3.17 |
| Fischpepton H-0230 | 5.45 | 8.22 | 3.30 |
| Fischpepton H-0430 | 5.88 | 12.3 | 4.77 |
| Fischpepton P-100 | 5.33 | 12.3 | 5.13 |
| Molkepulver, süss | 2.05 | 7.22 | 1.10 |
| Pepton C | 6.35 | 14.9 | 4.77 |
| Sojamehl, vollfett | n.b. | 2.62 | 1.10 |
| Sojamehl, entfettet | 5.95 | 8.423 | 2.57 |

| c) Versuch 3: synthetische N-Quellen, 16 h Anzucht: | | | |
|---|---|---|---|
| (NH₄)₂SO₄ | n.b. | 16.8 | 6.20 |
| NH₄Cl | n.b. | 14.0 | 3.79 |
| NH₄-Acetat | n.b. | 12.4 | 5.17 |
| L-Aspartat | n.b. | 10.0 | 3.10 |
| L-Asparagin | n.b. | 19.5 | 5.79 |
| L-Glutamat | n.b. | 15.3 | 4.76 |
| L-Lysin | n.b. | 11.0 | 2.66 |
| L-Prolin | n.b. | 17.8 | 4.83 |
| n.b.: nicht bestimmt | | | |

### c) Variation der Sorbitkonzentration

In Schüttelkulturen mit "Colominic Acid"-Produktionsmedium mit 5 g/l Hefeextrakt wird die Sorbitkonzentration in folgenden Schritten variiert: 0, 0.5, 1.25, 2.5, 5, 7.5 10.0 und 20.0 g/l. Der Versuch wird wie unter Beispiel 5a beschrieben durchgeführt.

Bei Sorbitkonzentrationen grösser als 2.5 g/l wird eine gute Enzymproduktion erreicht, das Optimum liegt bei 5-10 g/l.

### d) Variation der Hefeextraktkonzentration

Schüttelkulturversuche gemäss Beispiel 1a) mit "Colominic Acid"-Produktionmedium, welches 10 g/l Sorbit enthält, werden mit nachstehenden Hefeextraktkonzentrationen angesetzt: 0, 0.5, 1.0, 2.5, 5.0, 7.5 und 10.0 g/l.

Zwischen 1 und 10 g/l Hefeextrakt wird ein hoher Enzymgehalt im Rohextrakt gemessen, die höchsten Werte liegen bei 1 und 2.5 g/l.

### e) Variation des Start-pH-Wertes

Wachstum und CMP-Neu5Ac-Synthetaseproduktion werden, wie unter a) beschrieben, in Schüttelkulturen mit "Colominic Acid"-Produktionsmedium untersucht, wobei der Start-pH-Wert zwischen 5.5 und 10 in Schritten zu 0.5 Einheiten variiert.

Mehr als 50 % der maximalen Volumenausbeute (U/l Fermentationsbrühe) werden zwischen Start-pH 5.5 und 8.5 erzielt. Die Optima für die Volumenausbeute (6.0 U/l) und die spezifische Aktivität (20 mU/mg) liegen bei pH 6.5.

### Beispiel 2: Fermentative Gewinnung der Biomasse

### a) Fermentation unter Nachdosierung von Sorbit und Hefeextrakt

Zur Anzucht der Vorkulturen werden zwei 500 ml Erlenmeyerkolben mit je 100 ml der gemäss Beispiel 1 optimierten Nährlösung mit nachstehender Zusammensetzung durch Autoklavieren für 20 min. bei 120°C sterilisiert und mit Zellen von Escherichia coli K-235 (ATCC 13027), kultiviert auf einem geeigneten festen Nährmedium, beimpft.

Die Nährlösung hat die folgende Zusammensetzung:

| | |
|---|---|
| Sorbit | 10 g/l |
| Hefeextrakt | 2.5 g/l |
| K₂HPO₄ | 11 g/l |
| (NH₄)₂SO₄ | 2.5 g/l |
| MgSO₄×7H₂O | 1 g/l |
| pH-Wert | 6.5 |

Die Kulturen werden 15-16 h bei 37°C und 220 UpM geschüttelt.

10 l des o.g. Mediums, jedoch mit 6 g/l Sorbit, werden in einem Bioreaktor mit 15 l Gesamtvolumen durch Erhitzen auf 121°C für 30 min. sterilisiert. Die Schaumbekämpfung und die Regulation der Konzentration des gelösten Sauerstoffs sowie des pH-Wertes erfolgen automatisch über die Steuereinheit des Systems. Der pH-Wert wird durch Zugabe von 5 N H₃PO₄ und 5 N NaOH und die Konzentration an gelöstem Sauerstoff über die Rührerdrehzahl einreguliert. Die Betriebsparameter sind:

| | |
|---|---|
| Temperatur | 37°C |
| pH-Wert | 6.5 |
| gelöst Sauerstoffkonzentration | 50% Sättigung |
| maximale Rührerdrehzahl | 1200 UpM |
| maximale Belüftungsrate | 20 Nl/min = 2.0 VVM |
| (VVM: Volumen (Luft)/Volumen (Fermentergehalt)/min.) | |

Der Bioreaktor wird mit 150 ml Vorkultur angeimpft und in steril entnommenen Proben von Kulturbrühe wird alle 30 min. die Biomasse durch Messen der optischen Dichte bei 660 nm und der Enzymgehalt durch Bestimmung der CMP-Neu5Ac-Synthetase-Aktivität im Rohextrakt über die Zeit verfolgt (siehe Beispiel 1). Bei einer optischen Dichte von 3.8, 10.5 und 16.8 werden jeweils 400 ml einer sterilen Lösung mit 150 g/l Sorbit und 62.5 g/l Hefeextrakt unter aseptischen Bedingungen zugegeben. Die Biomasse wird nach Erreichen der maximalen Zelldichte durch Sedimentation in einer Durchflusszentrifuge abgetrennt und eingefroren bei -20°C gelagert.

Die maximal erreichte O.D.660 beträgt 20.8, entsprechend einem Feuchtmassegehalt von 45 g/l, und die maximale Enzymausbeute 28 U/l Fermentationsmedium, bei einer spez. Aktivität von 17 mU/mg Protein im Rohextrakt ohne Nukleinsäurefällung (vgl. Beispiel 3).

### b) Fermentation unter Nachdosierung von Sorbit, Hefeextrakt, MgSO₄ und Ammoniak

Im Vergleich mit Beispiel 2a) werden folgende Veränderungen vorgenommen:
- MgSO₄ wird als 100-fach konzentrierte Lösung getrennt von den anderen Mediumsbestandteilen autoklaviert,
- dem Medium werden 30 mg/l CaCl₂ zugesetzt,
- die pH-Regulation erfolgt mit 12.5 % Ammoniaklösung anstelle von 5 N NaOH,
- im Fermentationsverlauf werden Hefeextrakt und Sorbit in den o.g. Mengen 10 x nachgefüttert,
- bei einer Zelldichte von O.D.660 = 50 werden 70 ml einer sterilen MgSO₄-Lösung (100 g/l) zudosiert.

Es werden eine maximale O.D.660 von 70.3, ein Bakterienfeuchtmassegehalt von 195 g/l und eine Enzymvolumenausbeute von 67 U/l Fermentationsbrühe erzielt.

### Beispiel 3: Gewinnung des Rohextraktes

200 g Bakterienmasse (gemäss Beispiel 2 gewonnen) werden mit 300 ml einer Pufferlösung mit pH-Wert 9, bestehend aus 50 mM Tris/HCl, mit 1 mM Dithiothreitol und 20 mM MgCl₂ resuspendiert. Die Zellen werden solange in einem Ultraschallgerät unter Kühlung im Eisbad aufgeschlossen, bis über die Zeit keine weitere Zunahme der Protein-konzentration in der Lösung zu verzeichnen ist. Das Zellhomogenat wird mit 20.8 ml einer 10%igen Lösung von Polyethylenimin mit einer mittleren Molekülmasse von 6x10⁵ bis 1x10⁶, pH-Wert 9, versetzt, 15 min. im Eisbad unter gelegentlichem Rühren inkubiert, und anschliessend die präzipitierten Nukleinsäuren und ein Grossteil der Zelltrümmer durch Sedimentation für 60 min. bei 23000 x g und 4°C in einer Kühlzentrifuge abgetrennt. Zur weiteren Trubstoffentfernung wird der Rohextrakt für 30 min. bei 40000 x g und 4°C einem zweiten Zentrifugationsschritt unterworfen. Die Produktlösung (370 ml Gesamtvolumen) weist eine Volumenaktivität von 0.18 U/ml und eine spez. Aktivität von 9 mU/mg Protein auf. Im Vergleich mit Beispiel 2 wird ersichtlich, dass der Zellaufschluss mit Glasperlen höhere Synthetaseausbeuten ermöglicht als der Ultraschall-Aufschluss. Wie die Bestimmung der Reaktionsraten vor und nach der Polyethyleniminfällung zeigt, können die Nukleinsäuren mit diesem Schritt ohne Verlust an CMP-Neu5Ac Synthetase-Aktivität abgetrennt werden.

### Beispiel 4: Fermentative Gewinnung des CMP-Neu5Ac-Synthetase-haltigen Rohextraktes aus E.coli K-235 im 200 l Massstab

Abgesehen von nachstehenden Aenderungen wird wie unter Beispiel 2b verfahren, wobei, wenn nicht anders angegeben, eine generelle Massstabsvergrösserung um den Faktor 20 vorgenommen wird:
- 130 l Medium werden in einem 300l Fermenter sterilisiert und mit 1000 ml Vorkultur angeimpft.
- Die Belüftungsrate wird im Verlauf der Fermentation manuell dem Bedarf angepasst (0.5 - 1 VVM). Die Feinregelung der Konzentration an gelöstem Sauerstoff erfolgt durch automatische Variation der Drehzahl.
- Nach Erreichen einer O.D.660 von 4.3 werden während 14 h kontinuierlich 70 l einer zuvor autoklavierten konzentrierten Sorbit-/Hefeextraktlösung (159 g/l Sorbit und 66 g/l Hefeextrakt) unter sterilen Bedingungen zudosiert. Hierbei wird die Pumpgeschwindigkeit manuell so eingestellt, dass die Rührerdrehzahl und somit der stoffverbrauch gerade maximal sind.

Nach 26 h wird mit O.D.660= 40.3 die maximale Zelldichte erreicht. Der Fermenterinhalt wird mittels eines solehaltigen Kühlmittels auf 4°C abgekühlt, die Zellen in einer Durchflusszentrifuge sedimentiert und durch 2-malige Passage durch einen Hochdruck-Homogenisator aufgeschlossen. Nach dem Aufschluss beträgt die Enzymausbeute im Rohextrakt 8300 U mit einer spezifischen Aktivität von 12.2 mU/mg.

### Beispiel 5: Herstellung von Enzympräparationen mit angereicherter CMP-Neu5Ac-Synthetase Aktivität

### a) Fällung der CMP-Neu5Ac-Synthetase mit Polyethylenglycol

370 ml Rohextrakt (siehe Beispiel 3) werden mit 185 ml einer 50 %igen Lösung (w/w, Dichte = 1.08 kg/l) von Polyethylenglycol mit Molekulargewicht 6000 versetzt und der Ansatz über Nacht im Eisbad inkubiert. Das ausgefallene Protein wird durch Zentrifugation bei 4°C und 40000 x g für 1 h sedimentiert und das Pellet in 100 ml Enzympuffer (= 50 mM Tris/HCl, 1 mM Dithiothreitol, pH 7.5) gelöst. Das auf diese Weise gewonnene Enzympräparat weist eine spezifische Aktivität von 40 mU/mg und eine Volumenaktivität von 0.52 U/ml auf und ist weitgehend CTPase frei.

### b) Herstellung einer CMP-Neu5Ac-Synthetase-Präparation durch Batch-Adsorption an Butyl-Fraktogel TSK

450 ml eisgekühlter Rohextrakt werden mit 194 ml gesättigter Ammoniumsulfatlösung (= 100 %) und 600 ml Butyl-Fraktogel TSK (Fa. Merck, Darmstadt, FRG) vermischt, welches zuvor über einer G3-Sinterglasfritte mit 1.8 l einer 30 %igen Ammoniumsulfatlösung in Enzympuffer (siehe Beispiel 5a)) gewaschen worden ist. Nach 15 min. Inkubation bei 4°C unter leichtem Rühren wird das Gel über einer Glasfritte abgenutscht und mit 1.2 l einer 30 %igen sowie mit 1.8 l einer 15 %igen Ammoniumsulfatlösung in eisgekühltem Enzympuffer gewaschen. Die Elution erfolgt mit 1.8 l eisgekühltem Enzympuffer ohne Ammoniumsulfat. Das Eluat wird durch Ultrafiltration bei 4°C mittels eines Tangentialfluss-Systems Typ DC-2 über ein H1P30-43 Hohlfasermembran- Modul mit einem Abscheidegrad von 30000 Dalton (beides Fa. Grace, Division Amicon, Lausanne) auf 200 ml aufkonzentriert.

Die Konzentration der CMP-Neu5Ac-Synthetase im Retentat beträgt 0.12 U/ml bei einer spezifischen Aktivität von 11 mU/mg. Die Aufkonzentrierung durch Ultrafiltration verläuft ohne Verlust an Enzymaktivität. Das zu 43.5 % w/v mit Glycerin versetzte Enzympräparat kann bei -20°C über Monate ohne Aktivitätsverlust gelagert werden.

### c) Herstellung einer CMP-Neu5Ac-Synthetase-Präparation durch Batch-Adsorption an QAE-Sephadex

5 ml QAE-Sephadex A50 Gel werden mit 20 ml eisgekühltem Kaliumphosphat-Puffer, 50 mM, pH 7.5, in einem Filterröhrchen mit Porosität 1 gewaschen und mit 2.9 ml Rohextrakt (siehe Beispiel 3) unter leichtem Rühren für 15 min. bei 0°C zur Adsorption des Proteins inkubiert. Nach Absaugen der Proteinlösung wird das Gel 5 x mit je 5 ml des o.g. Phosphatpuffers gewaschen. Die Desorption des Proteins erfolgt mit Kochsalzlösungen ansteigender Konzentration (0.1, 0.13, 0.17, 0.2, 0.3 und 1 M) in genanntem Phosphatpuffer. Hierzu werden jeweils 12.5 ml der entsprechenden eisgekühlten NaCl-Lösung mit dem Gel vermischt, 5 min. bei 4°C inkubiert, abgesaugt und 3 ml des Eluates über eine 10 ml Gelfiltrationssäule (Econ-Pac 10DG, Fa. Biorad, Glattbrugg) entsalzt. Die Bestimmung der Proteinkonzentration und des Enzymgehalt ergibt, dass die Synthetase bei 0.2-0.3 M NaCl mit 33 % Ausbeute und einer mittleren spezifische Aktivität von 21 mU/mg von QAE-Sephadex desorbiert.

### d) Herstellung einer CMP -Neu5Ac -Synthetase-Präparation durch Batch-Adsorption an Hydroxylapatit

2 ml eines gemäss Beispiel 3 gewonnenen Rohextraktes werden mit 10 ml Hydroxylapatit (Fa. Biorad-Laboratories, Glattbrugg), welches zuvor in einer Glasfritte mit 30 ml Enzympuffer gewaschen worden ist, für 15 min. bei 4°C unter gelegentlichem Rühren inkubiert. Nach Absaugen der Probelösung wird das Gel mit 30 ml einer 100 mmolaren Lösung von Kaliumphosphat, pH-Wert 7.5, gespült. Die Desorption erfolgt mit 2 x 15 ml eisgekühltem 0.2 molarem und 1 x 15 ml 0.4 molarem Kaliumphosphatpuffer mit pH 7.5. Hierzu wird das abgenutschte Gel jeweils mit der entsprechende Pufferlösung versetzt und nach 10 min. Inkubation bei 4°C erneut über einer Glasfritte abgesaugL Die vereinigten Eluate werden mit einer Ultrafiltrationszelle Typ CEC1 über einer YM 30 Membran mit einem Abscheidegrad von 30000 Dalton (beides Fa. Grace, Division Amicon, Lausanne) auf 2 ml aufkonzentriert.

Die Aktivitäts- und Proteinbestimmung im Retentat, welches über eine Econ-Pac 10DG Gelfiltrationssäule ensalzt worden ist (4 ml Endvolumen), ergeben eine Volumenaktivität von 0.062 U/ml und eine spezifische Aktivität von 26 mU/mg.

### Beispiel 6: Synthese von CMP-Neu5Ac mit gemäss Beispiel 5 gewonnenen Enzympräparaten aus Escherichia coli K-235

### a) Variation der CTP- und der Neu5Ac-Konzentration

Die CTP- und die Neu5Ac-Konzentration (siehe Tabelle 3) werden in Ansätzen mit nachstehender Zusammensetzung variiert:

| | |
|---|---|
| Tris/HCl Puffer, pH 9 | 0.15 M |
| Dithiothreitol | 1.0 mM |
| CTP und Neu5Ac, je (beides Fa. Fluka, Buchs) | 5 - 50 mM |
| MgCl₂ | 40 - 65 mM |
| CMP-Neu5Ac Synthetase | 20 - 200 mU/ml |
| Gesamtvolumen | 250 µl |

Da im Verlauf der Reaktion Mg²⁺ mit Pyrophosphat als Magnesiumpyrophosphat ausfällt, wird bei Eduktkonzentrationen von 30 und 50 mM das MgCl₂ zu 15 mM im Überschuss zu den Edukten eingesetzt. Als Quelle der CMP-Neu5Ac-Synthetase dient ein gemäss Beispiel 5a) durch Fällung mit Polyethylenglycol hergestelltes Enzympräparat mit 0.71 U/ml Volumenaktivität und 34 mU/mg spezifischer Aktivität. Zum Umsatz von 1 mMol Edukt werden 4.7 U Synthetase eingesetzt. Nach 24 h Inkubation bei 30°C erfolgt die Aufarbeitung der Ansätze wie in Beispiel 1) beschrieben. Vor der Injektion auf die HPLC-Säule werden die Proben auf eine Edukt-Startkonzentration von 2 mM verdünnt.

Wie Tabelle 3 zeigt, wird die höchste CMP-Neu5Ac-Konzentration bei einer Konzentration der Edukte von jeweils 20 mM erreicht. Die molaren Ausbeuten nehmen mit zunehmender Eduktkonzentration ab. Durch Messung der Produktkonzentration im Zeitverlauf der Reaktion kann das Maximum der Ausbeute ermittelt und die Reaktion zum richtigen Zeitpunkt gestoppt werden.

**Tabelle 3:**

| Synthese von CMP-Neu5Ac in Abhängigkeit von der Konzentration der Edukte CTP und Neu5Ac | | | | |
|---|---|---|---|---|
| CTP/Neu5Ac (mM) | Enzymkonz. (mU/ml) | MgCl₂ (mM) | CMP-Neu5AC (mM) | molare Ausbeute (%) |
| 5 | 24 | 40 | 4.8 | 96 |
| 10 | 47 | 40 | 9.1 | 91 |
| 15 | 71 | 40 | 12.9 | 86 |
| 20 | 94 | 40 | 16.3 | 81 |
| 30 | 141 | 45 | 13.6 | 45 |
| 50 | 235 | 65 | 0.8 | 1.6 |

### b) Generierung von CTP aus CMP und ATP in situ

Die Synthese von CMP-Neu5Ac unter Generierung von CTP aus ATP und CMP wird mit 2 Enzympräparationen, die gemäss Beispiel 5 gewonnen worden sind, untersucht (siehe Tabelle 4). Alle Edukte werden zu je 10 mM eingesetzt und im weiteren wird wie unter Beispiel 6a) verfahren. Parallel werden Kontrollansätze mit CTP und Neu5Ac bereitet.

Wie aus Tabelle 4 ersichtlich, ist die Herstellung von CMP-Neu5Ac unter in situ-Generierung von CTP aus ATP und CMP mit den nach Beispiel 5b) und 5c) gewonnenen Enzymlösungen mit ca. 50 % der mit CTP erzielten Ausbeute möglich.

**Tabelle 4:**

| Synthese von CMP-Neu5Ac aus ATP, CMP und Neu5Ac (je zu 10 mM) | | | | |
|---|---|---|---|---|
| Enzympräparat/Beispiel | Enzymkonz. | pH-Wert | CMP-Neu5Ac aus | |
| | (mU/ml) | | ATP + CMP (mM) | CTP (mM) |
| Butyl-Fraktogel/ 5b) | 28 | 7.5 | 3.03 | 5.66 |
| Butyl-Fraktogel/ 5b) | 28 | 9 | 3.17 | 6.27 |
| QAE-Sephadex 5c) | 20 | 9 | 3.98 | 7.83 |

### c) Generierung von CTP aus Phosphoenolpyruvat und CMP in situ

Entsprechend der in Beispiel 6a) beschriebenen Verfahrensweise werden Syntheseansätze mit 25 mM Phosphoenolpyruvat und je 10 mM CMP und Neu5Ac bereitet. Die verwendeten Synthetase-Präparationen sind der Tabelle 5 zu entnehmen.

Sowohl mit dem Produkt der Batch-Adsorption an Butyl-Fraktogel als auch mit Rohextrakt ist die Synthese von CMP-Neu5Ac aus Phosphoenolpyruvat, CMP und Neu5Ac mit mehr als 50 % der Ausbeute via CTP möglich.

**Tabelle 5:**

| Synthese von CMP-Neu5Ac aus Phosphoenolpyruvat, CMP und Neu5Ac (10 mM) | | | | |
|---|---|---|---|---|
| Enzympräparat/gemäss Beispiel | Enzymkonz. | pH-Wert | CMP-Neu5Ac aus | |
| | (mU/ml) | | PEP^{*}) + CMP (mM) | CTP (mM) |
| Rohextrakt/ 3) | 35 | 7.5 | 4.19 | 6.7 |
| Rohextrakt/ 3) | 35 | 9 | 3.87 | 6.89 |
| Butyl-Fraktogel/5b) | 28 | 7.5 | 5.21 | 9.73 |
| Butyl/Fraktogel/5b) | 28 | 9 | 7.84 | 9.89 |

| | | | | |
|---|---|---|---|---|
| *): PEP: Phosphoenolpyruvat | | | | |

### d) Generierung von Neu5Ac aus Man2Ac und Pyruvat in situ

Die Synthese von CMP-Neu5Ac aus Man2Ac, Pyruvat und CTP durch die Kopplung einer Neuraminsäure-Aldolase mit der CMP-Neu5Ac-Synthetase-Reaktion wird in Abhängigkeit von der Eduktkonzentration untersucht. Da das pH-Optimum der Aldolase im neutralen Bereich liegt, wird die Reaktion bei pH 7.5 in Anwesenheit von MnCl₂ zur Aktivierung der Synthetase durchgeführt. Die Substrate Man2Ac und CTP werden in äquimolarem Verhältnis, Pyruvat in zweifachem und MnCl₂ zu 5 mM im Überschuss zu Man2Ac bzw. CTP eingesetzt. Als Enzyme werden verwendet eine Neu5Ac-Aldolasepräparation (Fa. Toyobo, Japan) und eine durch Fällung mit Polyethylenglycol gemäss Beispiel 5a) hergestellte Enzymlösung mit Synthetase-Aktivität. Die Konzentration der Aldolase (spezifische Aktivität 8 U/mg) beträgt 0.5 mg und die der Synthetase 4.7 U, jeweils pro 1 mMol umzusetzendes Man2Ac bzw. CTP. Die Durchführung des Versuchs erfolgt wie unter Beispiel 6a) beschrieben.

Wie Tabelle 6 zeigt, steigen die Produktkonzentration und die molare Ausbeute mit der Eduktkonzentration auf maximal 18.4 mM bzw. 37 % an.

**Tabelle 6:**

| Synthese von CMP-Neu5Ac aus Man2Ac, Pyruvat und CTP unter Katalyse von Neu5Ac-Aldolase und CMP-Neu5Ac Synthetase | | | | |
|---|---|---|---|---|
| Man2Ac/CTP (mM) | Pyruvat (mM) | MgCl₂ (mM) | CMP-Neu5AC (mM) | molare Ausbeute^{* )} (%) |
| 5 | 10 | 10 | 0.52 | 10 |
| 10 | 20 | 15 | 2.3 | 23 |
| 15 | 30 | 20 | 5.06 | 34 |
| 20 | 40 | 25 | 6.62 | 30 |
| 30 | 60 | 35 | 11.1 | 37 |
| 50 | 100 | 55 | 18.4 | 37 |

| | | | | |
|---|---|---|---|---|
| *): molaren Ausbeuten bezogen auf Man2Ac bzw. CTP | | | | |

### e) Generierung von Neu5Ac aus N-Acetylglucosamin und Pyruvat in situ

Bei der Synthese von CMP-Neu5Ac aus N-Acetylglucosamin, Pyruvat und CTP wird das N-Acetylglucosamin mittels einer N-Acetylglucosamin-Epimerase zu Man2Ac epimerisiert. Diese Verbindung kondensiert mit Pyruvat unter Katalyse der im Beispiel 6d) verwendeten Neu5Ac-Aldolase zu Neu5Ac.

Der Reaktionsansatz mit 250 µl Geamtvolumen beinhaltet:

| | |
|---|---|
| Tris/HCl-Puffer | 150 mM |
| MnCl₂ | 55 mM |
| Dithiothreitol | 1 mM |
| CTP | 50 mM |
| Natriumpyruvat | 100 mM |
| N-Acetylglucosamin | 200 mM |
| N-Acetylglucosamin-Epimerase (Fa. Toyobo, Japan) | 300 mU/ml |
| Neu5Ac-Aldolase | 200 mU/ml |
| CMP-Neu5Ac-Synthetase | 235 mU/ml |
| pH-Wert | 7.1 |

Als Quelle der Synthetase wird ein durch Fällung mit Polyethylenglycol gewonnenes Enzympräparat (siehe Beispiel 5a) verwendet. Im weiteren wird gemäss Beispiel 6a) verfahren.

Nach 20 h Inkubation bei 30°C wird mittels HPLC eine CMP-Neu5Ac-Konzentration von 5.3 mM entsprechend einem Umsatz von ca. 10 %, bezogen auf CTP, bestimmt.

### f) Präparative Synthese von CMP-Neu5Ac als Reinsubstanz

Ein Ansatz nachstehender Zusammensetzung wird für 20 h bei 30°C in einem Wasserbad unter leichtem Rühren inkubiert:

| | |
|---|---|
| Tris/HCl, pH 9 | 150 mM |
| MgCl₂ | 40 mM |
| Dithiothreitol | 1 mM |
| Neu5Ac | 15 mM |
| CTP | 15 mM |
| CMP-Neu5Ac Synthetasepräparation gemäss Beispiel 5a) | 7.04 % v/v (entsprechend 0.05 U/ml Endkonz.) |
| pH-Wert | 9, eingestellt mit verdünnter NaOH |
| Gesamtvolumen | 150 ml |

Alle Schritte zur Aufarbeitung des CMP-Neu5Ac werden bei 0 - 4°C durchgeführt. Ausfällungen werden durch Zentrifugation für 20 min. bei 25000 x g abgetrennt, in 150 ml einer 10 mmolaren NH₃-Lösung resuspendiert und erneut abzentrifugiert. Nach Wiederholung dieses Waschschrittes wird das Protein aus den vereinigten Überständen abgetrennt mittels Ultrafiltration über eine YM 30 Membran (Abscheidegrenze 30000 Dalton, Fa. Grace, Division Amicon, Lausanne) in einem CEC1 -Durchflusskonzentrator des selben Herstellers. Bei einem Retentatvolumen von 15 ml wird dieses mit 15 ml NH₃-Lösung ( 1 mM) verdünnt und erneut auf 15 ml aufkonzentriert. Dieser Diafiltrationsschritt wird zweimal wiederholt.

Das Filtrat (480 ml) wird mit einer Flussrate von 25 ml/h auf eine 5 x 25 cm Dowex 1X8-Säule (200 - 400 mesh, Bicarbonat-Form) aufgetragen, welche zuvor mit 2 Säulenvolumen 1 mM NH₃ gespült worden ist. Anschliessend wird die Säule mit 1 l NH₃-Lösung (1mM) nachgewaschen. Die Elution erfolgt mit o.g. Flussrate an einem linearen Gradienten (2 x 5 l) von 0.01 - 1 M Triethylammoniumhydrogencarbonat, pH 8.4. Die CMP-Neu5Ac enthaltenden Fraktionen werden mittels Dünnschichtchromatographie (siehe Beispiel 1 ) identifiziert, vereinigt und lyophilisiert.

Die Abtrennung von Verunreinigungen mit CMP erfolgt mittels Gelchromatographie an Sephadex G-25 (Fa. Pharmacia). Hierzu werden 0.29 g des Lyophilisates in 16 ml 1 mM NH₃-Lösung gelöst und die Probelösung mit einer Flussrate von 70 ml/h über die Säule (5 x 80 cm) gefördert, welche zuvor mit 4.5 NH₃-Lösung (1 mM) äquilibriert worden ist. Zur Aufarbeitung des gesamten Produktes aus der Anionenaustauschchromatographie wird der Gelfiltrationsschritt 3 mal durchgeführt. Diejenigen Fraktionen, welche gemäss dünnschichtchromatographischer Analyse nur CMP-Neu5Ac enthalten, werden vereinigt und gefriergetrocknet. Es werden 0.79 g des Ammoniumsalzes von CMP-Neu5Ac erhalten.

### Beispiel 7: Abtrennung der Zelltrümmer mittels wäßriger Zweiphasen-Systeme

In Vorversuchen wird pH = 7,50 und T = 10°C als optimal festgestellt. Das Zellhomogenat wird mittels Mikrowelle aufgetaut, darf die Temperatur von 10°C aber nicht überschreiten. Das Zellhomogenat und die abzentrifugierten Phasensysteme werden stets im Eisbad gehalten.

Das Polymer PEG mit einem Molekulargewicht von 1550 (PEG-1550) wird in verschiedenen Endkonzentrationen von 16,87 - 17,61 % w/w mit Tri-Natriumcitrat x 6 H₂O in Endkonzentrationen zu 9,69 - 9,84 % w/w sowie mit dem Zellhomogenat (ZH) aus E.coli K-235 (47,65 - 46,04 % w/w Endkonzentration) zusammengemischt. Hierbei wird das Tri-Natriumcitrat x 6 H₂O als 40 %ige (w/v) Lösung eingesetzt, deren pH-Wert zuvor mit einer 40 %igen (w/v) Zitronensäure-Lösung auf 7,50 eingestellt worden ist. Mit deionisiertem Wasser werden die Ansätze auf 100 % Gesamtgewicht (= 10 - 600 g) aufgefüllt und 5 min. lang manuell oder mit einem Schüttler geschüttelt. Anschliessend werden die Phasensysteme 20 min. bei 8000 UpM in einer Kühlzentrifuge bei 4°C abzentrifugiert.

Folgende Zusammensetzungen erweisen sich für die Synthetase-Extraktion als optimal:
Zusammensetzung 1: 16,87 % w/w PEG-1550 + 9,69 w/w % Tri-Natriumcitrat x 6 H₂O + 47,65 w/w % ZH,
Zusammensetzung 2: 17,61 % w/w PEG- 1550 + 9, 84 w/w % Tri-Natriumcitrat x 6 H₂O + 46,04 w/w % ZH

In die Oberphase eines 10 g Phasensystems mit Zusammensetzung 1 (5.4 ml, 1,75 mg Protein/ml) werden mit einer Ausbeute von 96 % 2,7 U Synthetase extrahiert, in die Oberphase eines 10 g System mit Zusammensetzung 2 (5.3 ml, 1.71 mg Protein/ml) 2,32 U entsprechend einer Ausbeute von 90 %.

Die entstandenen zwei Phasen werden getrennt, in dem die PEG-1550 angereicherte Oberphase sauber von der Unterphase abdekantiert wird. Die Oberphase, die die CMP-Neu5Ac -Synthetase und PEG-1550 enthält, wird für die weitere Aufreinigung mittels Hydrophobe-Interaktions-Chromatographie (HIC) eingesetzt.

Zur Ermittlung der Phosphatase-Aktivität werden folgende Stammlösungen hergestellt: das Reagenz = 10 mM 4-Nitrophenylphosphat (PNPP, Fa. Serva) mit pH 8,0, 7 mM MgCl₂ in 100 mM Tris-HCl, pH 8,0 und 100 mM Tris-HCl, pH 8,0.

Für die Bestimmung der Phosphatase-Aktivität werden 10 µl der zu untersuchenden Probe mit 100 µl PNPP, 10 µl MgCl₂ und 880 µl Tris-HCl-Puffer versetzt. Die Extinktion wird bei 401 nm direkt sowie nach 20 min Inkubation bei 30°C gemessen. Die Bestimmung der Konzentrationen an freigesetztem 4-Nitrophenol erfolgt an Hand einer Standardlösung von 4-Nitrophenol. Folgende Phosphatase-Aktivitäten werden ermittelt (1 U = 1 µMol gebildetes 4-Nitrophenol/min.):

| Probe | Phosphatase-Aktivität (U/ml) |
|---|---|
| Zellhomogenat | 0,347-0,353 |
| Oberphase | 0,04-0,09 |
| Unterphase | 0,142-0,162 |
| HIC-Fraktion/Durchlauf (vgl. Beispiel 8) | 0,0013 |
| HIC-Fraktion/Synthetase (vgl. Beispiel 8) | 0,003 |

### Beispiel 8: Hydrophobe Interaktions-Chromatographie

### a) Aufreinigung der Synthetase mittels Hydrophobe Interaktions- Chromatographie (HIC) (A)

Probe: 3 ml Oberphase aus Beispiel 7 mit der Synthetase; Matrix: Phenyl-Sepharose CL-4B. Firma: Pharmacia Kapazität: 15-20 mg HSA (Human-Serumalbumin)/ml Gel. Säule: 26 mm Durchmesser x 4 cm Höhe =ca. 50 ml Gelbett. Durchflußrate: 1,53 ml/min. Eluent A: 50 mM Na₂HPO₄ + 1,8 M (NH₄)₂SO₄, pH 7,0. Eluent B: 50 mM Na₂HPO₄, pH 7,0. Gradient: 0-100 % B in 2,5 h, linearer Gradient. Detektion: UV, 280 nm

Nachdem die Säule mit mindestens drei Bettvolumina 3 M Harnstoff-Lösung gereinigt, anschließend mit Wasser gespült und mit dem Puffer A equilibriert worden ist, werden 200 ml der synthetase-haltige Oberphase auf die Phenyl-Sepharose CL-4B Säule aufgetragen. Anschließend wird solange mit Puffer A gespült, bis alles ungebundene Protein von der HIC-Säule abgespült ist. Danach eluiert man mit einen linearen Gradienten von 0 % B bis 100 % B mit 250 ml A und 250 ml B. Dabei wird die lonenstärke erheblich reduziert. Nach dem Gradient spült man mit Eluent B über 30 min und anschließend mit Wasser, um die Synthetase von der Säule zu desorbieren. Die Elution wird mit einem UV-Monitor von der Firma Pharmacia bei 280 nm verfolgt. 14 U Synthetase aus 20 ml der Oberphase mit einer Protein-Konzentration von 13,58 mg/ml werden mit 98,87 % Wiederfindungsrate aufgereinigt.

### b) Aufreinigung der Synthetase mit HIC (B)

Probe: PEG-1550-haltige Oberphase mit der Synthetase. Matrix: Phenyl-Sepharose CL-4B. Kapazität: 15-20 mg HSA pro ml Gel. Säule: 26 mm Durchmesser x 4 cm Höhe =ca. 50 ml Gelbett. Durchflußrate: 1,53 ml/min. Puffer A: 50 mM Na₂HPO₄ + 1,8 M (NH₄)₂SO₄, pH 7,0. Puffer B: 50 mM Na₂HPO₄, pH 7,0. Gradient: 0-100 % B in 2,5 h, Stufengradient. Detektion: UV, 280 nm

In diesem Versuch werden die Bedingungen von Beispiel 8a angewendet mit dem Unterschied, daß man hier mit einem Stufengradient eluiert. Die Ausbeute beträgt hier etwa 85 %.

### c) Aufreinigung der Synthetase mit HIC (C)

Probe: PEG-1550-haltige Oberphase mit der Synthetase. Matrix: Phenyl-Sepharose CL-4B. Kapazität: 15-20 mg HSA pro ml Gel. Säule: 26 mm Durchmesser x 4 cm Höhe =ca. 50 ml Gelbett. Durchflußrate: 1,53 ml/min. Puffer A:50 mM Na₂HPO₄ + 1,8 M (NH₄)₂SO₄, pH 7,0. Puffer B: 50 mM Na₂HPO₄, pH 7,0. Elutionsmittel: 10 % v/v Ethylenglycol in Wasser. Gradient: 0-100 % B in 2,5 h, linearer Gradient. Detektion:UV, 280 nm

In diesem Fall wird so wie im Beispiel 8a vorgegangen. Die Synthetase wird hier nicht mit Wasser, sondern mit einem Gemisch Wasser/Ethylenglycol von der hydrophoben Säule desorbiert. Die Ausbeute ist hier vergleichbar mit der von Beispiel 8a. Für die späteren Arbeiten wird daher das Eluieren der Synthetase allein mit Wasser bevorzugt.

### d) Scale up der Hydrophoben Interaktions-Chromatographie

Versuch: Aufreinigung der Synthetase mit HIC. Probe: PEG-1550-haltige Oberphase mit der Synthetase. Matrix: Phenyl-Sepharose CL-4B. Kapazität: 15-20 mg HSA/ml Gel. Säule: 113 mm Durchmesser x 16 cm Betthöhe = ca. 1,521 Liter Gelbett. Durchflußrate: 7,72 ml/min. Puffer A: 50 mM Na₂HPO₄ + 1,5 M (NH₄)₂SO₄, pH 7,0. Puffer B:50 mM Na₂HPO₄, pH 7,0. Gradient: 0-100 % B in 8 h, linearer Gradient. Detektion: UV Absorption bei 280 nm.

Hier wird ebenfalls wie im Beispiel 8a vorgegangen, jedoch ist die Flußrate höher. 140 U Synthetase aus 200 ml der Oberphase mit einer Protein-Konzentration von 15,58 mg/ml werden mit 95 % Wiederfindungsrate aufgereinigt. Die Packungsqualität der Säule wird in diesem Fall getestet und beträgt 2817 N/m (Theoretische Böden pro Quadratmeter).

### Beispiel 9: Kontinuierliche Synthese von CMP-Neu5Ac aus CTP und Neu5Ac im Enzymmembranreaktor

Die Synthese von CMP-Neu5Ac aus CTP und Neu5Ac kann kontinuierlich in einem Enzymmembranreaktor (EMR) mit einer Ultrafiltrationsmembran mit einem Abscheidegrad kleiner gleich 30000 Dalton durchgeführt werden, welcher die CMP-Neu5Ac Synthetase enthält, in den die Edukte CTP und Neu5Ac eingespeist werden und aus dessen Auslauf CMP-Neu5Ac isoliert und aufgereinigt wird.

Im EMR können die Enzyme in löslicher Form angewandt werden, da sie darin durch die Ultrafiltrationsmembran zurückgehalten werden, während das niedermolekulare Produkt und nicht umgesetzte Edukte diese ungehindert passieren. Der Reaktor wird vor Betriebsbeginn sterilisiert, so dass auf den Zusatz antibakterieller Substanzen verzichtet werden kann. Die das Edukt enthaltene Lösung wird dem Reaktor sterilfiltriert über einen Mikrofilter zugeführt.

Der Zulauf hat die folgende Zusammensetzung:

| | |
|---|---|
| Tris/HCl-Puffer, pH 8 | 100 mM |
| MgCl₂ | 15 mM |
| Neu5Ac | 10 mM |
| CTP | 10 mM |

Mit verdünnter Natronlauge wird ein pH-Wert von 8 eingestellt. Die Zulauflösung wird im Eisbad gekühlt bevorratet.

Ein mit Hilfe der HIC (vgl. Beispiel 8) aufgereinigtes Synthetasepräparat wird in einer Endkonzentration von 1U Synthetase/ml Reaktorvolumen als Katalysator eingesetzt.

Die folgenden Betriebsbedingungen werden während des 24 stündigen Versuchs eingehalten:

| | |
|---|---|
| Temperatur | 25°C |
| Reaktorvolumen | 10 ml |
| Verweilzeit | 1 h |

Im Reaktorauslauf wird eine Konzentration von CMP-Neu5Ac von 6 mM gemessen. Das gebildete CMP-Neu5Ac wird, wie in Beispiel 6f beschrieben, mittels Diafiltration, Anionenaustauscherchromatographie und Gelchromatographie gereinigt.

### Beispiel 10: Kontinuierliche Synthese von CMP-Neu5Ac aus CTP und Neu5Ac im Enzymmembranreaktor mit pH-Regulation

Abgesehen von nachstehenden Aenderungen wird wie unter Beispiel 9 beschrieben verfahren:

Zur Regulation des pH-Wertes im Reaktor wird ein handelsüblicher ph-Stat eingesetzt. Die Messung des Ist-Wertes erfolgt im Auslauf mittels einer Durchlaufelektrode unmittelbar hinter der Ultrafitrationsmembran des Reaktors. Die Korrekturlösung (1N NaOH) wird dem Zulauf direkt vor dem Sterilfilter zugeführt. Beim Aufbau der Apparatur werden die Totvolumina zwischen Sterilfilter und Reaktor sowie Reaktor und pH-Durchflusselektrode möglichst klein gehalten.

Im Reaktorauslauf wird eine Konzentration von CMP-Neu5Ac von 7,52 mM gemessen, was einem Umsatz von 75% und einer Raum-Zeit-Ausbeute von 111 g/l Reaktorvolumen/Tag entspricht. Das gebildete CMP-Neu5Ac wird, wie in Beispiel 6f beschrieben, mittels Diafiltration, Anionenaustauscherchromatographie und Gelchromatographie gereinigt.

### Beispiel 11: Selektionierung und Charakterisierung einer Mutante (CS1) von E. coli K-235 mit erhöhter Produktivität

### a) Gewinnung der Mutante CS1

Zellmasse von E. coli K-235 (ATCC 13027) wird auf handelsüblichem Plate Count Agar (1.0 g/l Glucose, 5.0 g/l Trypton, 2.5 g/l Hefeextrakt, pH-Wert ca. 7), supplementiert mit 7 g/l Agar, mittels einer Impföse zur Zellvereinzelung ausgestrichen. Die Platten werden zunächst für 24 h bei 37°C und anschliessend für 2-4 Wochen bei Raumtemperatur bebrütet. Nach diesem Zeitraum beginnen die Kolonien grossflächig auszuwachsen und zu verschleimen. Nicht schleimiges Zellmaterial, welches am Rand schleimiger Kolonien seitlich hervorwächst, wird abgeimpft und in üblicher Weise durch Zellvereinzelung mittels Ausstreichen in Reinkultur gebracht.

Die Koloniemorphologie des so gewonnenen Stammes CS1 unterscheidet sich von der des Ausgangsorganismus K-235 wie folgt:
- Nach Wachstum bei 37°C und anschliessend bei 28°C für jeweils 24 h erscheinen die Kolonien der neuen Zellinie im Durchlicht leicht transparent und weisen einen unregelmässig gezackten Rand auf. Die Kolonien der Ausgangskultur hingegen sind nicht durchscheinend, etwas dicker, gelblicher und weisen einen glatten Rand auf.
- bei längerer Inkubation, z. B. länger als ca. eine Woche bei 28°C, bildet die neue Kultur keine schleimigen Kolonien.

### b) Produktion der CMP-Neu5Ac Synthetase durch Stamm CS1 und E. coli K-235 im komplexen Medium sowie im Mineralsalzmedium

Zur weiteren Charakterisierung des neuen Stammes CS1 werden das Wachstum und die CMP-Neu5Ac Synthetaseproduktivität im Vergleich mit dem Ursprungsstamm in Schüttelkultur gemessen. Die Anzucht erfolgt im Hefeextrakt/Sorbitmedium (siehe Beispiel 2a) mit 10g/l Sorbit bzw. im nachstehenden Mineralsalzmedium:

| | |
|---|---|
| Sorbit | 10.0 g/l |
| KH₂PO₄ | 7.81 g/l |
| (NH₄)₂HPO₄ | 2.33 g/l |
| CaCl₂ x 2 H₂O | 0.03 g/l |
| MgSO₄ x 7 H₂O (Lösung (392 g/l)) | 10.0 ml/l |
| Thiaminhydrochlorid-Lösung (2.67 g/l) | 1.0 ml/l |
| Spurenelementlösung | 10.0 ml/l |
| Fe^{III}-Citrat Lösung, (0.75 g/l) | 50.0 ml/l |
| pH-Wert | 6.5 |

Die Zusammensetzung der Spurenelementlösung ist:

| | |
|---|---|
| CoCl₂ x 6 H₂O | 0.267 g/l |
| MnCl₂ x 4 H₂O | 1.6 g/l |
| CuCl₂ x 2 H₂O | 0.151 g/l |
| H₃BO₃ | 0.333 g/l |
| Na₂MoO₄ x 2 H₂O | 0.267 g/l |
| ZnSO₄ x 7 H₂O | 1.093 g/l |

Sorbit, KH₂PO₄, (NH₄)₂HPO₄ und CaCl₂ x 2 H₂O werden in 93 % des Endvolumens angesetzt. Nach dem Autoklavieren der salzhaltigen Lösungen werden diese unter sterilen Bedingungen vereinigt. Die Thiaminlösung wird sterilfiltriert zugegeben. Die weitere Vorgehensweise ist unter Beispiel la angegeben.

Wie Tabelle 7 zeigt, wachsen beide Organismen auf dem Mineralsalzmedium wie auf dem komplexen Medium zu jeweils gleicher Zelldichte heran. Die Enzymaktivität von Stamm CS1 ist in beiden Medien deutlich höher als die von E. coli K-235.

**Tabelle 7:**

| Vergleich des Wachstums und der Enzymproduktion von Variante CS1 und E. coli K-235 im komplexen und im Mineralsalz-Medium | | | | | | |
|---|---|---|---|---|---|---|
| Medium | | Mikroorganismus | | | | |
| | E. coli K-235 | | | Stamm CS1 | | |
| | Zelldichte (O.D.660) | spez. Aktivität (mU/mg) | Volumenausbeute (U/l) | Zelldichte (O.D.660) | spez. Aktivität (mU/mg) | Volumen ausbeute (U/l) |
| Sorbit/ Hefeextrakt | 5.45 | 17.3 | 9.48 | 5.51 | 26.7 | 13.1 |
| Sorbit/ Mineralsalze | 3.58 | 16.9 | 7.21 | 3.56 | 32.9 | 12.9 |

### b) Einfluss verschiedener Kohlenstoffquellen auf das Wachstum der E. coli Stämme CS1 und K-235 und auf die Produktion der CMP-Neu5Ac Synthetase

Wie in Beispiel 1 beschrieben,wird der Einfluss verschiedener C-Quellen zu 10 g/l auf das Wachstum und die CMP-Neu5Ac Synthetaseaktivität getestet. Als Grundmedium dient die gemäss Beispiel 2a zur Fermentation verwendete Nährlösung (Hauptunterschiede zu Beispiel 1a: 2.5 anstatt 0.5 g/l Hefeextrakt und pH 6.5 statt 7.8).

Aus Tabelle 8 ist ersichtlicht, dass die Stämme CS1 und K-235 das gleiche Spektrum zur Verwertung von C-Quellen aufweisen. Insbesonders mit Sorbit und Na-Acetat werden hohe Enzymausbeuten erzielt.

**Tabelle 8:**

| Vergleich des Wachstums und der Enzymproduktion von Stamm CS1 und E.coli K-235 in Anwesenheit verschiedener C-Quellen | | | | | | |
|---|---|---|---|---|---|---|
| C-Quelle | | | Mikroorganismus | | | |
| | E. coli K-235 | | | Stamm CS1 | | |
| | Zelldichte (O.D.660) | spez. Aktivität (mU/mg) | Volumenausbeute (U/l) | Zelldichte (O.D.660) | spez. Aktivität (mU/mg) | Volumen ausbeute (U/l) |
| Xylose | 5.72 | 7.0 | 2.92 | 6.14 | 14.8 | 8.61 |
| Sorbit | 5.74 | 17.7 | 7.93 | 5.65 | 27.2 | 13.5 |
| Na-Succinat | 2.28 | 5.6 | 1.82 | 2.5 | 8.0 | 2.96 |
| Saccharose | 1.36 | 1.5 | 0.34 | 1.34 | 3.6 | 0.87 |
| Na-Acetat | 3.27 | 23.7 | 9.50 | 3.59 | 43.8 | 16.8 |
| Äpfelsäure | 2.7 | 10.7 | 3.17 | 2.6 | 23.0 | 7.67 |
| Milchsäure | 3.7 | 28.0 | 10.6 | 3.6 | 38.7 | 15.8 |
| n.b.: nicht bestimmt | | | | | | |

### Beispiel 12: Fermentative Gewinnung der Biomasse von Zellvariante CS1

Zur fermentativen Gewinnung der Biomasse von Stamm CS1 wird im wesentlichen gemäss Beispiel 2 b) verfahren. Die Vorgehensweise wird wie folgt abgeändert:
- Es wird ein 19 l Bioreaktor mit 7l Füllstand eingesetzt.
- Sorbit und Hefeextrakt werden in 21 Portionen zu je 140 ml nachgefüttert. Die Zusammensetzung der ersten 13 Portionen ist: 300 g/l Sorbit, 125 g/l Hefeextrakt, 11 g/l K₂HPO₄, 2.5 g/l (NH₄)₂SO₄, 30 mg/l CaCl₂. Für die restlichen Zudosierungen werden Sorbit und Hefeextrakt in o.g. Konzentration in deionisiertem Wasser gelöst.
- Bei einer Zelldichte von O.D.660 = 88 wird der Fermentationsbrühe K₂HPO₄ zu einer Endkonzentration von 1.5 g/l zudosiert.

Zum Zeitpunkt der maximalen O.D.660 (= 94.0) werden ein Biofeuchtmassegehalt von 314 g/l (Trockenmasse = 50.9 g/l) und eine Enzymvolumenausbeute von 191 U/l Fermentationsbrühe bei einer spezifischen Aktivität von 28.0 mU/mg erreicht.

Der Stamm E.coli K-235/CS1 kann an Stelle des Stammes E.coli K-235 für die Gewinnung von Zellextrakten mit Aktivität an CMP-Neu5Ac-Synthetase verwendet werden, welche wiederum erfindungsgemäss für die Herstellung von CMP-aktivierten Sialinsäuren verwendet werden können, in Analogie zu den vorangehenden Beispielen.

### Hinterlegung von Mikroorganismen

Der Stamm E.coli K-235/CS1 wurde bei der Deutschen Sammlung von Mikroorganismen (DSM), Mascheroder Weg 1b, D-3300 Braunschweig, am 18.6. 1992 unter der Hinterlegungsnummer DSM 7114 hinterlegt.

## Patentansprüche

1. Verfahren zur Herstellung von Cytidin-5'-monophospho-sialinsäuren, dadurch gekennzeichnet, daß man
a) unter natürlich vorkommenden Mikroorganismen einen solchen identifiziert, der Cytidin-5'-monophospho-N-acetylneuraminsäure-Synthase-Aktivität (CMP-Neu5AC-Synthetase-Aktivität) aufweist, den identifizierten Mikroorganismus kultiviert, die Zellen von der Kulturbrühe in an sich bekannter Weise separiert, ihre Zellwände aufbricht, die unlöslichen Bestandteile verwirft und den verbleibenden flüssigen
Zellextrakt roh weiterverwendet oder vorher lediglich einem Reinigungsschritt unterzieht, und b) man eine Sialinsäure in Gegenwart des Zellextraktes gemäß (a) mit Cytidin-5'-triphosphat umsetzt.

2. Verfahren nach Anspruch 1, dadurch gekennzeichnet, daß der Zellextrakt gemäß Stufe (a) dadurch gewonnen wird, daß die den Mikroorganismus mit Aktivität an CMP-Neu5Ac-Synthetase enthaltene Kulturbrühe zentrifugiert wird, die Zellen in an sich bekannter Weise aufgeschlossen werden und b) man eine Sialinsäure in Gegenwart des gemäß (a) gewonnenen Überstands der Zentrifugation des Zellhomogenats mit Cytidin-5'-triphosphat umsetzt.

3. Verfahren gemäss Anspruch 1, dadurch gekennzeichnet, daß der Zellextrakt gemäß Stufe (a) dadurch gewonnen wird, daß die den Mikroorganismus mit Aktivität an CMP-Neu5Ac-Synthetase enthaltene Kulturbrühe zentrifugiert wird, die Zellen in an sich bekannter Weise aufgeschlossen werden und die Zelltrümmer mittels eines wässrigen Zweiphasensystems abgetrennt werden, und b) man eine Sialinsäure in Gegenwart des Zellextraktes gemäß (a) mit Cytidin-5'-triphosphat umsetzt.

4. Verfahren nach einem der Ansprüche 2 oder 3, dadurch gekennzeichnet, daß man den Zellextrakt gemäß Stufe (a) zuerst einem Reinigungsschritt ausgewählt aus den Reinigungs-schritten Fällung mit Polyäthylenglykol, Anionenaustauscherchromatographie, hydrophobe Interaktionschromatographie, Gelfiltration, Affinitätschromatographie, Chromatographie an Hydroxylapatit und Chromatofokussierung unterwirft, bevor man ihn in Stufe (b) einsetzt.

5. Verfahren nach Anspruch 1, dadurch gekennzeichnet, daß der Zellextrakt gemäß Stufe (a) dadurch gewonnen wird, daß die den Mikroorganismus mit Aktivität an CMP-Neu5Ac-Synthetase enthaltene Kulturbrühe zentrifugiert wird, die Zellen in an sich bekannter Weise aufgeschlossen werden, die Zelltrümmer mittels eines wässrigen Zweiphasensystems abgetrennt werden, der Ueberstand mittels hydrophober Interaktions-Chromatographie gereinigt wird, und b) man eine Sialinsäure in Gegenwart des gemäß (a) gewonnenen mit Cytidin-5'-triphosphat umsetzt.

6. Verfahren nach Anspruch 1, dadurch gekennzeichnet, daß die Reaktion im Batch-Verfahren durchgeführt wird.

7. Verfahren nach Anspruch 1, dadurch gekennzeichnet, daß die Reaktion kontinuierlich im Enzymmembranreaktor durchgeführt wird.

8. Verfahren nach Anspruch 1, dadurch gekennzeichnet, daß die Umsetzung in homogener wässriger Lösung bei 20-35 C in Gegenwart von etwa 10-50 mM Mg²⁺ oder in Gegenwart von etwa 5-30 mM Mn²⁺ durchgeführt wird, wobei der pH-Wert der Reaktionsmischung bei Anwesenheit von Mg²⁺-lonen etwa 8-11 und bei Anwesenheit von Mn²⁺-lonen etwa 6-8 beträgt.

9. Verfahren zur Herstellung von CMP-Neu5Ac nach Anspruch 7, dadurch gekennzeichnet, daß die Umsetzung von Neu5Ac mit CTP gemäß Stufe (b) kontinuierlich in einem Enzymmembranreaktor, welcher den gegebenenfalls einem Reinigungsschritt unterworfenen Zellextrakt gemäß Stufe (a) eines natürlich vorkommenden Mikroorganismus mit Aktivität an CMP-Neu5Ac-Synthetase enthält, in den die Edukte Neu5Ac und CTP eingespeist werden und aus dessen Auslauf CMP-Neu5Ac isoliert wird, durchgeführt wird.

10. Verfahren zur Herstellung von CMP-Neu5Ac nach Anspruch 5, dadurch gekennzeichnet, daß es sich bei dem Mikroorganismus mit CMP-Neu5Ac-Synthetase-Aktivität gemäß Stufe (a) um E.coli K-235 (ATCC13027) handelt.

11. Verfahren zur Herstellung von CMP-Neu5Ac nach Anspruch 5, dadurch gekennzeichnet, daß es sich bei dem Mikroorganismus mit CMP-Neu5Ac-Synthetase-Aktivität gemäß Stufe (a) um E-coli K-235/CS1 (DSM 7114) handelt.

12. Verfahren nach einem der Ansprüche 1 bis 11, dadurch gekennzeichnet, daß die Sialinsäure gemäß Stufe (b) ausgewählt wird aus der Gruppe bestehend aus N-Acetylneuraminsäure (Neu5Ac), N-Acetyl-4-O-acetylneuraminsäure (Neu4,5Ac₂), N-Acetyl-9-O-acetylneuraminsäure ((Neu5,9Ac₂), N-Acetyl-7,9-di-O-acetylneuraminsäure (Neu5,7,9Ac₃), N-Acetyl-8,9-di-O-acetylneuraminsäure (Neu5,8,9Ac₃), N-Acetyl-9-O-lactoylneuraminsäure (Neu4Ac9Lt), N-Acetyl-4-O-acetyl-9-O-lactoylneuraminsäure (Neu4,5Ac₂9Lt), N-Acetylneuraminsäure-9-phosphat (Neu5Ac9P), N-Glycoloylneuraminsäure (Neu5Gc), N-Glycoloyl-9-O-acetylneuramin-säure (Neu9Ac5Gc), N-Glycoloyl-9-O-lactoylneuraminsäure (Neu5Gc9Lt), N-Glycoloyl-neuraminsäure-8-sulfat ((Neu5Gc8S), 5-Azidoneuraminsäure, N-Acetyl-9-azido-9-deoxy-neuraminsäure oder N-Acetyl-9-acetamido-9-deoxy-neuraminsäure.

13. Verfahren nach Anspruch 12, dadurch gekennzeichnet, daß die Sialinsäure gemäß Stufe (b) ausgewählt wird aus der Gruppe bestehend aus Neu4,5Ac₂, Neu5,9Ac₂, Neu5Gc, Neu9Ac5Gc, N-Acetyl-9-acetamido-9-deoxy-neuraminsäure, N-Acetyl-9-azido-9-deoxy--neuraminsäure, 5-Azidoneuraminsäure und Neu5Ac.

14. Verfahren nach Anspruch 13, dadurch gekennzeichnet, daß daß die Sialinsäure gemäß Stufe (b) CMP-Neu5Ac ist.

15. Verfahren nach Anspruch 1, dadurch gekennzeichnet, daß die Sialinsäure gemäß Stufe (b) im Reaktionsgemisch aus geeigneten Vorstufen in situ bereitgestellt wird.

16. Verfahren nach Anspruch 1, dadurch gekennzeichnet, daß Cytidin-5'-triphosphat (CTP) gemäß Stufe (b) im Reaktionsgemisch aus geeigneten Vorstufen in situ bereitgestellt wird

17. Verfahren nach einem der Anspruche 1 bis 11, dadurch gekennzeichnet, daß in Stufe (a) ein Zellextrakt eines natürlich vorkommenden Mikroorganismus ausgewählt aus der Gruppe bestehend aus Escherichia coli Serotyp K1, einer Spezies von Streptococcus Serotyp B oder C, Neisseria meningitidis, Pasteurella haemolytica, Pasteurella multacida, Moraxella nonliquefaciens, Citrobacter freundii, einer Salmonella-Spezies, z.B. S. dahlem, S, djakarta oder S.- trouca, Actinomyces viscosus oder Corynebacterium parvum verwendet wird.

18. Verfahren nach Anspruch 17, dadurch gekennzeichnet, daß in Stufe (a) ein Zellextrakt eines E.coli-Stammes des Serotyps K1 verwendet wird.

19. Verfahren nach Anspruch 18, dadurch gekennzeichnet, daß in Stufe (a) ein Zellextrakt von E coli K-235 (ATCC 13027) verwendet wird.

20. Verfahren nach Anspruch 18, dadurch gekennzeichnet, daß ein Zellextrakt von E.coli K-235/CS1 (DSM 7114) verwendet wird.

21. E.coli K-235/CS1 (DSM 7114) mit Cytidin-5'-monophospho-N-acetylneuraminsäureSynthase-Aktivität.

## Claims

1. A process for the preparation of a cytidine-5'-monophosphosialic acid which comprises a) identifying from naturally occurring microorganisms a microorganism that has cytidine5'-monophospho-N-acetylneuraminic acid synthetase activity (CMP-Neu5Ac-synthetase activity), culturing the identified microorganism, separating the cells in a manner known per se from the culture broth, breaking open their cell walls, discarding the insoluble components and using the residual liquid cell extract in crude form or subjecting it beforehand to only one purification step, and b) reacting a sialic acid with cytidine-5'-triphosphate in the presence of the cell extract according to (a).

2. A process according to claim 1, wherein the cell extract according to step (a) is obtained by centrifuging the culture broth containing the microorganism having CMP-Neu5Ac-synthetase activity and breaking open the cells in a manner known per se and b) a sialic acid is reacted with cytidine-5'-triphosphate in the presence of the supernatant obtained according to (a) from the centrifugation of the cell homogenate.

3. A process according to claim 1, wherein the cell extract according to step (a) is obtained by centrifuging the culture broth containing the microorganism having CMP-Neu5Ac-synthetase activity, breaking open the cells in a manner known per se, and removing the cell debris by means of an aqueous two-phase system, and b) a sialic acid is reacted with cytidine-5'-triphosphate in the presence of the cell extract according to (a).

4. A process according to either claim 2 or claim 3, wherein the cell extract according to step (a) is first subjected to a purification step selected from precipitation with polyethylene glycol, anion exchange chromatography, hydrophobic interaction chromatography, gel filtration, affinity chromatography, chromatography on hydroxy apatite and chromatofocussing before being used in step (b).

5. A process according to claim 1, wherein the cell extract according to step (a) is obtained by centrifuging the culture broth containing the microorganism having CMP-Neu5Ac-synthetase activity, breaking open the cells in a manner known per se, removing the cell debris by means of an aqueous two-phase system, and purifying the supernatant by means of hydrophobic interaction chromatography, and b) a sialic acid is reacted with cytidine-5'-triphosphate in the presence of the supernatant obtained according to (a).

6. A process according to claim 1, wherein the reaction is carried out in a batch process.

7. A process according to claim 1, wherein the reaction is carried out continuously in an enzyme membrane reactor.

8. A process according to claim 1, wherein the reaction is carried out in homogeneous aqueous solution at 20-35°C in the presence of approximately 10-50 mM Mg²⁺ or in the presence of approximately 5-30 mM Mn²⁺, the pH value of the reaction mixture being approximately 8-11 in the presence of Mg²⁺ ions and approximately 6-8 in the presence of Mn²⁺ ions.

9. A process for the preparation of CMP-Neu5Ac according to claim 7, wherein the reaction of Neu5Ac with CTP according to step (b) is carried out continuously in an enzyme membrane reactor that contains the cell extract according to step (a) of a naturally occurring microorganism having CMP-Neu5Ac-synthetase activity, which cell extract has optionally been subjected to a purification step, into which reactor the starting materials Neu5Ac and CTP are fed and from the outflow of which reactor CMP-Neu5Ac is isolated.

10. A process for the preparation of CMP-Neu5Ac according to claim 5, wherein the microorganism having CMP-Neu5Ac synthetase activity according to step (a) is E. coli K-235 (ATCC 13027).

11. A process for the preparation of CMP-Neu5Ac according to claim 5, wherein the microorganism having CMP-Neu5Ac synthetase activity according to step (a) is E. coli K-235/CS1 (DSM 7114).

12. A process according to any one of claims 1 to 11, wherein the sialic acid according to step (b) is selected from the group consisting of N-acetylneuraminic acid (Neu5Ac), N-acetyl-4-O-acetylneuraminic acid (Neu4,5Ac₂), N-acetyl-9-O-acetylneuraminic acid (Neu5,9Ac₂), N-acetyl-7,9-di-O-acetylneuraminic acid (Neu5,7,9Ac₃), N-acetyl-8,9-di-O-acetylneuraminic acid (Neu5,8,9Ac₃), N-acetyl-9-O-lactoylneuraminic acid (Neu4Ac9Lt), N-acetyl-4-O-acetyl-9-O-lactoylneuraminic acid (Neu4,5Ac₂9Lt), N-acetylneuraminic acid 9-phosphate (Neu5Ac9P), N-glycoloylneuraminic acid (Neu5Gc), N-glycoloyl-9-O-acetylneuraminic acid (Neu9Ac5Gc), N-glycoloyl-9-O-lactoylneuraminic acid (Neu5Gc9Lt), N-glycoloylneuraminic acid 8-sulfate (Neu5Gc8S), 5-azidoneuraminic acid, N-acetyl-9-azido-9-deoxy-neuraminic acid and N-acetyl-9-acetamido-9-deoxy-neuraminic acid.

13. A process according to claim 12, wherein the sialic acid according to step (b) is selected from the group consisting of Neu4,5Ac₂, Neu5,9Ac₂, Neu5Gc, Neu9Ac5Gc, N-acetyl-9-acetamido-9-deoxy-neuraminic acid, N-acetyl-9-azido-9-deoxy-neuraminic acid, 5-azidoneuraminic acid and Neu5Ac.

14. A process according to claim 13, wherein the sialic acid according to step (b) is CMP-Neu5Ac.

15. A process according to claim 1, wherein the sialic acid according to step (b) is prepared in situ in the reaction mixture from suitable precursors.

16. A process according to claim 1. wherein the cytidine-5'-triphosphate (CTP) according to step (b) is prepared in situ in the reaction mixture from suitable precursors.

17. A process according to any one of claims 1 to 11, wherein in step (a) a cell extract of a naturally occurring microorganism selected from the group consisting of Escherichia coli serotype K1, a species of Streptococcus serotype B or C, Neisseria meningitidis, Pasteurella haemolytica, Pasteurella multacida, Moraxella nonliquefaciens, Citrobacter freundii, a Salmonella species, e.g. S. dahlem, S. djakarta or S. trouca, Actinomyces viscosus and Corynebacterium parvum is used.

18. A process according to claim 17, wherein in step (a) a cell extract of an E. coli strain of the serotype K1 is used.

19. A process according to claim 18, wherein in step (a) a cell extract of E. coli K-235 (ATCC 13027) is used.

20. A process according to claim 18. wherein a cell extract of E. coli K-235/CS 1 (DSM 7114) is used.

21. E. coli K-235/CS1 (DSM 7114) having cytidine-5'-monophospho-N-acetylneuraminic acid synthetase activity.

## Revendications

1. Procédé pour la préparation d'acides cytidine-5'-monophospho-sialiques, caractérisé en ce que
a) parmi les micro-organismes d'origine naturelle, on en identifie un qui a une activité cytidine-5'-monophospho-acide-N-acétylneuramique-synthétase (activité de CMP-Neu5Ac-synthétase), on cultive le micro-organisme identifié, on sépare les cellules du bouillon de culture de manière connue en soi, on brise leurs parois cellulaires, on rejette les constituants insolubles et on utilise l'extrait cellulaire liquide restant à l'état brut ou bien on le soumet simplement à une opération de purification, et
b) on fait réagir un acide sialique avec le cytidine-5'-triphosphate en présence de l'extrait cellulaire obtenu en a).

2. Procédé selon revendication 1, caractérisé en ce que l'on obtient l'extrait cellulaire du stade (a) en centrifugeant le bouillon de culture contenant le micro-organisme ayant l'activité de CMP-Neu5Ac-synthétase, on brise les cellules de manière connue en soi, et
(b) on fait réagir un acide sialique avec le cytidine-5'-triphosphate en présence du surnageant de la centrifugation de l'homogénéisat cellulaire obtenu en (a).

3. Procédé selon revendication 1, caractérisé en ce que l'on obtient l'extrait cellulaire du stade (a) en centrifugeant le bouillon de culture contenant le micro-organisme ayant l'activité de CMP-Neu5Ac-synthétase, en brisant les cellules de manière connue en soi et en séparant les débris cellulaires par un système aqueux à deux phases, et
(b) on fait réagir un acide sialique avec le cytidine-5'-triphosphate en présence de l'extrait cellulaire obtenu en (a).

4. Procédé selon une des revendications 2 ou 3, caractérisé en ce que l'on soumet d'abord l'extrait cellulaire obtenu au stade (a) à une opération de purification choisie parmi la précipitation par un polyéthylèneglycol, la chromatographie d'échange d'anions, la chromatographie d'interaction hydrophobe, la filtration sur gel, la chromatographie d'affinité, la chromatographie sur hydroxylapatite et la chromatofocalisation, avant de le mettre en oeuvre au stade (b).

5. Procédé selon revendication 1, caractérisé en ce que l'on obtient l'extrait cellulaire du stade (a) en centrifugeant le bouillon de culture contenant le micro-organisme ayant une activité de CMP-Neu5Ac-synthétase, en brisant les cellules de manière connue en soi, en séparant les débris cellulaires par un système aqueux à deux phases, en purifiant le liquide surnageant par chromatographie d'interaction hydrophobe et
(b) on fait réagir un acide sialique avec le cytidine-5'-triphosphate en présence du produit obtenu en (a).

6. Procédé selon la revendication 1, caractérisé en ce que la réaction est réalisée en discontinu.

7. Procédé selon revendication 1, caractérisé en ce que la réaction est exécutée en continu dans un réacteur enzymatique à membrane.

8. Procédé selon revendication 1, caractérisé en ce que la réaction est réalisée en solution aqueuse homogène à une température de 20 à 35°C en présence d'environ 10 à 50 mM de Mg⁺² ou en présence d'environ 5 à 30 mM de Mn⁺², le pH du mélange de réaction étant d'environ 8 à 11 dans le cas de la présence d'ions Mg⁺² et d'environ 6 à 8 dans le cas de la présence d'ions Mn⁺².

9. Procédé de préparation du CMP-Neu5Ac selon revendication 7, caractérisé en ce que la réaction entre le Neu5Ac et le CTP du stade (b) est réalisée en continu dans un réacteur enzymatique à membrane qui contient l'extrait cellulaire, éventuellement soumis à une opération de purification du stade (a), d'un micro-organisme d'origine naturelle ayant une activité de CMP-Neu5Ac-synthétase, dans lequel on introduit les produits de départ Neu5Ac et CTP et de l'effluent duquel on isole le CMP-Neu5Ac.

10. Procédé de préparation du CMP-Neu5Ac selon revendication 5, caractérisé en ce que le micro-organisme à activité de CMP-Neu5Ac-synthétase du stade (a) est E. Coli K-235 (ATCC 13027).

11. Procédé de préparation du CMP-Neu5Ac selon revendication 5, caractérisé en ce que le micro-organisme ayant une activité de CMP-Neu5Ac-synthétase du stade (a) est E. Coli K -235/CS1 (DSM 7114).

12. Procédé selon une des revendications 1 à 11, caractérisé en ce que l'acide sialique du stade (b) est choisi dans le groupe consistant en l'acide N-acétylneuramique (Neu5Ac), l'acide N-acétyl-4-O-acétylneuramique (Neu4,5Ac₂), l'acide N-acétyl-9-0-acétylneuramique (Neu5, 9Ac₂), l'acide N-acétyl-7,9-di-O-acétylneuramique (Neus,7,9Ac₃), l'acide N-acétyl-8,9-diO-acétylneuramique (Neu5, 8,9Ac₃), l'acide N-acétyl-9-0-lactoylneuramique (Neu4Ac9Lt), l'acide N-acétyl-4-0- acétyl-9-0-lactoylneuramique (Neu4, 5Ac₂9Lt), le 9-phosphate de l'acide N-acétylneuramique (Neu5Ac9P), l'acide N-glycoloylneuramique (Neu5Gc), l'acide N-glycoloyl-9-0-acétylneuramique (Neu9Ac5Gc), l'acide N-glycoloyl-9-0-lactoylneuramique (Neu5Gc9Lt), le 8-sulfate de l'acide N-glycoloylneuramique (Neu5Gc8S), l'acide 5-azidoneuramique, l'acide N-acétyl-9-azido-9-désoxyneuramique ou l'acide N-acétyl-9-acétamido-9-désoxyneuramique.

13. Procédé selon revendication 12, caractérisé en ce que l'acide sialique du stade (b) est choisi dans le groupe consistant en le Neu4,5Ac₂, le Neu5,9Ac₂, le Neu5Gc, le Neu9Ac5Gc, l'acide N-acétyl-9-acétamido-9-désoxyneuramique, l'acide N-acétyl-9-azido-9-désoxyneuramique, l'acide 5-azidoneuramique et le Neu5Ac.

14. Procédé selon revendication 13, caractérisé en ce que l'acide sialique du stade (b) est le CMP-Neu5Ac.

15. Procédé selon revendication 1, caractérisé en ce que l'acide sialique du stade (b) est préparé in situ dans le mélange de réaction à partir de produits intermédiaires appropriés.

16. Procédé selon revendication 1, caractérisé en ce que le cytidine-5'-triphosphate (CTP) du stade (b) est préparé in situ dans le mélange de réaction à partir de produits intermédiaires appropriés.

17. Procédé selon une des revendications 1 à 11, caractérisé en ce que, au stade (a), on utilise un extrait cellulaire d'un micro-organisme d'originel naturelle choisi dans le groupe consistant en Escherichia coli, sérotype K1, une espèce de Streptococcus, sérotype B ou C, Neisseria meningitidis, Pasteurella haemolytica, Pasteurella multacida, Moraxella nonliquefaciens, Citrobacter freundii, une espèce Salmonella, par exemple S. dahlem, S. djakarta ou S. trouca, Actinomyces viscosus ou Corynebacterium parvum.

18. Procédé selon revendication 17, caractérisé en ce que, au stade (a), on utilise un extrait cellulaire d'une souche d'E. Coli du sérotype K1.

19. Procédé selon revendication 18, caractérisé en ce que, au stade (a), on utilise un extrait cellulaire d'E. Coli K-235 (ATCC 13027).

20. Procédé selon revendication 18, caractérisé en ce que l'on utilise un extrait cellulaire d' E. Coli K-235/CS1 (DSM 7114).

21. E. Coli K-235/CS1 (DSM 7114), ayant une activité de cytidine-5'-monophospho-acide N-acétylneuramique-synthétase.
